# EUROPEAN PATENT APPLICATION

(11) **EP 4 813 302 A1**
(43) Date of publication of application: **30.09.2026**
(21) Application number: 24894222.9
(22) Date of filing: 22.11.2024
(51) Int. Cl.: A61B 6/00, A61B 6/08, A61B 6/46

(54) **X-RAY IMAGING DEVICE**

(30) Priority: 24.11.2023 JP 2023199366; 19.02.2024 JP 2024023082
(71) Applicant: SHIMADZU CORPORATION, Kyoto-shi, Kyoto 604-8511 (JP)
(72) Inventor: FURUHASHI, Naoya, Kyoto-shi, Kyoto 604-8511 (JP); NAKATSU, Kohei, Kyoto-shi, Kyoto 604-8511 (JP); OKUMURA, Hiroshi, Kyoto-shi, Kyoto 604-8511 (JP); TAKEDA, Ryo, Kyoto-shi, Kyoto 604-8511 (JP)
(74) Representative: Müller-Boré & Partner Patentanwälte PartG mbB
(86) International application number: PCT/JP2024/041408
(87) International publication number: WO 2025/110232

(57) **Abstract**

This X-ray imaging apparatus (100) includes an optical imaging unit (31) capturing an optical image (70), and an optical imaging control unit (32). The optical imaging control unit is configured to perform control to switch from a first mode of displaying the optical image superimposed with a region display (80) including at least one of a detection unit region display (81), an X-ray irradiation field region display (84), and an X-ray phototiming field region display (87) with reference to a predetermined fixed position in a real space, or displaying the optical image without superimposing the region display, to a second mode of displaying the optical image superimposed with the region display with reference to a position on a body surface (101a) of a subject (101) in the real space.

## Description

### TECHNICAL FIELD

The present invention relates to an X-ray imaging apparatus, and more particularly to an X-ray imaging apparatus including an optical imaging unit.

### BACKGROUND ART

Conventionally, an X-ray imaging apparatus including an optical imaging unit has been known. Such an X-ray imaging apparatus is disclosed in, for example, U.S. Patent Application Publication No. 2021/0150704.

The above U.S. Patent Application Publication No. 2021/0150704 describes an imaging system (X-ray imaging apparatus). This imaging system includes an X-ray source, an X-ray detector, a camera, and a display unit. The camera is arranged facing a patient. Further, in the imaging system disclosed in the above U.S. Patent Application Publication No. 2021/0150704, when position adjustment of the patient and the X-ray source is performed, a video image of the patient captured by the camera is displayed on the display unit. Further, in the imaging system disclosed in the above U.S. Patent Application Publication No. 2021/0150704, a frame line corresponding to a region where X-rays emitted from the X-ray source are detected by the X-ray detector is superimposed on the video image of the patient and displayed on the display unit. At this time, the frame line corresponding to the region detected by the X-ray detector is deformed into a shape corresponding to a body surface of the patient, and displayed on the body surface of the patient.

### Citation List

### Patent Literature

Patent Literature 1: U.S. Patent Application Publication No. 2021/0150704

### SUMMARY OF THE INVENTION

### PROBLEMS TO BE SOLVED BY THE INVENTION

However, when a frame line (region display) corresponding to a region where an X-ray irradiated from an X-ray source is detected by an X-ray detector is deformed into a shape corresponding to a body surface of a patient (subject) and displayed as in the imaging system (X-ray imaging apparatus) of the above-mentioned US Patent Application Publication No. 2021/0150704, the position and shape of the region display change every time the position of the body surface of the subject changes. When performing position adjustment of the subject and the X-ray source (X-ray irradiation unit), fine adjustment of positions of the subject and the X-ray irradiation unit is performed after performing rough position adjustment of the subject and the X-ray irradiation unit. During the rough position adjustment of the subject and the X-ray irradiation unit, the position of the subject significantly changes. When the position of the subject significantly changes, the position and shape of the region display are frequently changed, making it difficult to confirm the position of the subject in a video image (optical image) displayed on a display unit. As a result, the work efficiency of the position adjustment decreases. Therefore, there is a demand for an X-ray imaging apparatus capable of easily performing position adjustment while suppressing a decrease in work efficiency of position adjustment.

The present invention has been made to solve the above-described problems, and one object of the present invention is to provide an X-ray imaging apparatus capable of easily performing position adjustment while suppressing a decrease in work efficiency of position adjustment.

### MEANS FOR SOLVING THE PROBLEMS

An X-ray imaging apparatus according to a first aspect of the present invention includes an X-ray irradiation unit including an X-ray tube, an X-ray detection unit detecting an X-ray irradiated from the X-ray irradiation unit and transmitted through a subject, an optical imaging unit capturing an optical image of the subject, a display unit displaying the optical image captured by the optical imaging unit, a control unit performing control to display the optical image on the display unit, and an input receiving unit receiving an input from an operator, and the control unit is configured to, when performing position adjustment, perform control to switch from a first mode in which, based on the input from the operator received by the input receiving unit, the optical image superimposed with a region display including at least one of a detection unit region display indicating a region where the X-ray detection unit is arranged in the optical image, an X-ray irradiation field region display indicating a region irradiated with the X-ray by the X-ray irradiation unit in the optical image, and an X-ray phototiming field region display indicating a region for phototiming the X-ray irradiated by the X-ray irradiation unit in the optical image with reference to a predetermined fixed position in a real space is displayed, or the optical image in which the region display is not superimposed on the optical image and the region display is hidden is displayed, to a second mode in which the optical image superimposed with the region display with reference to a position on a body surface of the subject in the real space is displayed. Note that the predetermined fixed position in the real space is a position set in advance in an irradiation axis direction of the X-ray in a state where the X-ray irradiation unit and the X-ray detection unit face each other.

Also, an X-ray imaging apparatus according to a second aspect of the present invention includes an X-ray irradiation unit including an X-ray tube, an X-ray detection unit detecting an X-ray irradiated from the X-ray irradiation unit and transmitted through a subject, an optical imaging unit capturing an optical image of the subject, a display unit displaying the optical image captured by the optical imaging unit, a body surface position acquisition unit acquiring a position of a body surface of the subject in a real space, a control unit performing control to display the optical image on the display unit, and an input receiving unit receiving an input from an operator, and the control unit is configured to, when performing position adjustment, perform control to switch from a first mode in which, based on the input from the operator received by the input receiving unit or the position of the body surface of the subject acquired by the body surface position acquisition unit, the optical image superimposed with a region display including at least one of a detection unit region display indicating a region where the X-ray detection unit is arranged in the optical image, an X-ray irradiation field region display indicating a region irradiated with the X-ray by the X-ray irradiation unit in the optical image, and an X-ray phototiming field region display indicating a region for phototiming the X-ray irradiated by the X-ray irradiation unit in the optical image with reference to a predetermined fixed position in the real space is displayed, or the optical image in which the region display is not superimposed on the optical image and the region display is hidden is displayed, to a second mode in which the optical image superimposed with the region display with reference to a position on the body surface of the subject in the real space is displayed.

### EFFECTS OF THE INVENTION

The X-ray imaging apparatus according to the first aspect and the second aspect includes the control unit configured to, when performing position adjustment as described above, perform control to switch from the first mode in which the optical image superimposed with the region display with reference to the predetermined fixed position in the real space is displayed based on the input from the operator, or the optical image in which the region display is not superimposed on the optical image and the region display is hidden is displayed, to the second mode in which the optical image superimposed with the region display with reference to the position on the body surface of the subject in the real space is displayed.

Thereby, when displaying the optical image superimposed with the region display in the first mode, the optical image superimposed with the region display at the fixed position is displayed, so that even if the position of the body surface of the subject changes when performing the rough position adjustment of the subject and the X-ray irradiation unit, the region display can be fixedly displayed. Therefore, it is possible to prevent the position and shape of the region display from changing, so that it is possible to suppress a decrease in work efficiency of position adjustment caused by frequent changes in the position and shape of the displayed region display when performing the rough position adjustment of the subject and the X-ray irradiation unit. Also, when the optical image in which the region display is not superimposed and the region display is hidden is displayed in the first mode, the region display is not superimposed on the optical image, so that it is possible to prevent a decrease in work efficiency of position adjustment caused by frequent changes in the position and shape of the displayed region display when performing the rough position adjustment of the subject and the X-ray irradiation unit. Also, after the rough position adjustment of the subject and the X-ray irradiation unit, the operator performs fine adjustment of positions of the subject and the X-ray irradiation unit. In the fine adjustment of the positions of the subject and the X-ray irradiation unit, the positions of the subject and the X-ray irradiation unit are adjusted such that a region of the X-ray irradiated on the body surface of the subject comes to a position corresponding to a detection region of the X-ray detection unit. In the second mode switched from the first mode based on the input from the operator, the optical image superimposed with the region display on the body surface of the subject is displayed. Therefore, when performing the fine adjustment of the positions of the subject and the X-ray irradiation unit, the operator can easily perform the position adjustment while visually recognizing the region display superimposed on the body surface of the subject in the optical image. As a result of these, it is possible to provide an X-ray imaging apparatus capable of easily performing position adjustment while suppressing a decrease in work efficiency.

Also, the X-ray imaging apparatus according to the second aspect includes, as described above, the body surface position acquisition unit acquiring the position of the body surface of the subject in the real space, and the control unit performing control to display the optical image on the display unit, and the control unit performs control to switch from the first mode in which, based on the position of the body surface of the subject acquired by the body surface position acquisition unit when performing position adjustment, the optical image superimposed with the region display with reference to the predetermined fixed position in the real space is displayed, or the optical image in which the region display is not superimposed on the optical image and the region display is hidden is displayed, to the second mode in which the optical image superimposed with the region display with reference to the position on the body surface of the subject in the real space is displayed. Thereby, since switching from the first mode to the second mode is performed based on the position of the body surface of the subject acquired by the body surface position acquisition unit, switching from the first mode to the second mode can be automatically performed without the operator performing an operation input. As a result, it is possible to provide an X-ray imaging apparatus capable of easily performing position adjustment while achieving both reduction in work burden on the operator and suppression of a decrease in work efficiency.

### BRIEF DESCRIPTION OF THE DRAWINGS

[FIG. 1] FIG. 1 is a schematic diagram showing an overall configuration of an X-ray imaging apparatus according to a first embodiment.
[FIG. 2] FIG. 2 is a schematic diagram showing a configuration of a holding unit according to the first embodiment.
[FIG. 3] FIG. 3 is a block diagram showing the overall configuration of the X-ray imaging apparatus according to the first embodiment.
[FIG. 4] FIG. 4 is a schematic diagram showing a configuration of an optical image generated by an optical imaging control unit.
[FIG. 5] FIG. 5 is a schematic diagram for explaining an optical image in which a region display including a detector region display, an X-ray irradiation field region display, and an AEC region display is superimposed on the optical image.
[FIG. 6] FIG. 6 is a schematic diagram for explaining an optical image displayed in a first mode.
[FIG. 7] FIG. 7 is a schematic diagram for explaining an optical image displayed in a second mode.
[FIG. 8] FIG. 8 is a schematic diagram (1) for explaining calculation of an X-ray tube-to-subject distance by the optical imaging control unit of the X-ray imaging apparatus according to the first embodiment.
[FIG. 9] FIG. 9 is a schematic diagram (2) for explaining calculation of the X-ray tube-to-subject distance by the optical imaging control unit of the X-ray imaging apparatus according to the first embodiment.
[FIG. 10] FIG. 10 is a flowchart for explaining a control process of an image display method by the X-ray imaging apparatus.
[FIG. 11] FIG. 11 is a block diagram showing an overall configuration of an X-ray imaging apparatus according to a second embodiment.
[FIG. 12] FIG. 12 is a schematic diagram for explaining a configuration in which an optical imaging control unit according to the second embodiment switches a mode for displaying a region display from a second mode to a first mode.
[FIG. 13] FIG. 13 is a flowchart for explaining a configuration in which the optical imaging control unit according to the second embodiment switches the mode for displaying the region display from the second mode to the first mode.
[FIG. 14] FIG. 14 is a block diagram showing an overall configuration of an X-ray imaging apparatus according to a third embodiment.
[FIG. 15] FIG. 15 is a flowchart for explaining a control process of an image display method by the X-ray imaging apparatus according to the third embodiment.
[FIG. 16] FIG. 16 is a schematic diagram (1) for explaining calculation of an X-ray tube-to-subject distance by an optical imaging control unit of an X-ray imaging apparatus according to a modification.
[FIG. 17] FIG. 17 is a schematic diagram (2) for explaining calculation of the X-ray tube-to-subject distance by the optical imaging control unit of the X-ray imaging apparatus according to the modification.

### DESCRIPTION OF EMBODIMENTS

Hereinafter, embodiments embodying the present invention will be described with reference to the drawings.

### [First Embodiment]

### (Configuration of X-ray Imaging Apparatus)

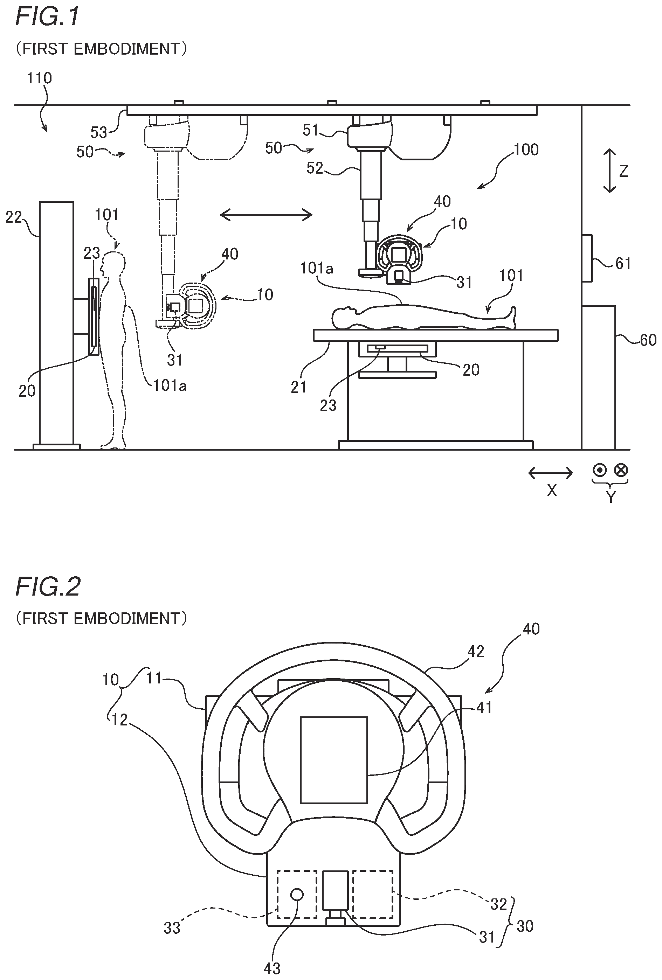

With reference to Figs. 1 to 8, a configuration of an X-ray imaging apparatus 100 according to a first embodiment of the present invention will be described.

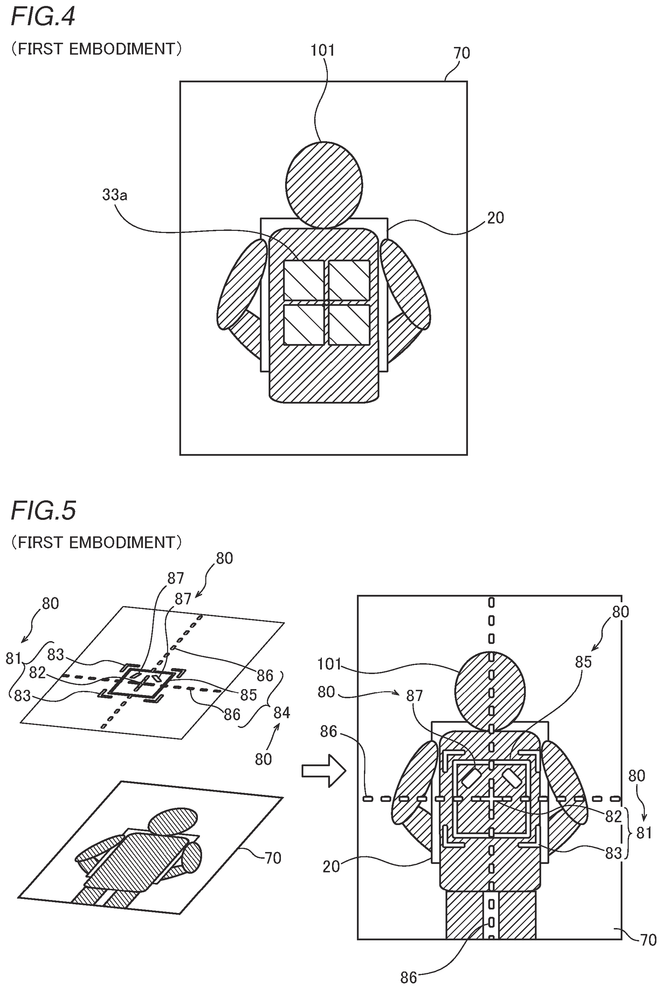

Fig. 1 shows an example of a ceiling-suspended X-ray imaging apparatus 100. The X-ray imaging apparatus 100 includes an X-ray irradiation unit 10, an X-ray detection unit 20, an optical imaging unit 31, a holding unit 40, a moving mechanism 50, an apparatus control unit 60, and an input unit 61. The X-ray imaging apparatus 100 is an imaging apparatus including a medical X-ray imaging apparatus, and is configured to perform X-ray imaging of a subject 101 which is an imaging target. In the X-ray imaging apparatus 100, X-rays irradiated from the X-ray irradiation unit 10 are detected by the X-ray detection unit 20, whereby X-ray imaging of the subject 101 is performed. Further, in the X-ray imaging apparatus 100, an optical image 70 (see Fig. 4) obtained by imaging the appearance of the subject 101 is captured by the optical imaging unit 31. Moreover, in the X-ray imaging apparatus 100, the X-ray irradiation unit 10, the X-ray detection unit 20, the optical imaging unit 31, the holding unit 40, and the moving mechanism 50 are installed in an imaging room 110, while the apparatus control unit 60 and the input unit 61 are installed outside the imaging room 110.

In the ceiling-suspended X-ray imaging apparatus 100, the holding unit 40 holding the X-ray irradiation unit 10 is held so as to be suspended from the ceiling by the moving mechanism 50 arranged on the ceiling of the imaging room 110. The holding unit 40 is held movably within the imaging room 110 by the moving mechanism 50. Note that a vertical (perpendicular) direction is defined as a Z direction, and two directions orthogonal to each other in a horizontal direction are defined as an X direction and a Y direction.

The X-ray imaging apparatus 100 includes an imaging table 21 for performing imaging in a posture in which the subject 101 is laid down (decubitus posture), and an imaging stand 22 for performing imaging in a posture in which the subject 101 stands up (upright posture). The X-ray detection unit 20 is movably held by each of the imaging table 21 and the imaging stand 22. The X-ray detection unit 20 includes, for example, a flat panel detector (FPD). The X-ray detection unit 20 is configured to detect X-rays that are irradiated from the X-ray irradiation unit 10 and have passed through the subject 101. The moving mechanism 50 can move the holding unit 40 at least between an imaging position in the decubitus posture using the imaging table 21 (see solid lines in Fig. 1) and an imaging position in the upright posture using the imaging stand 22 (see two-dot chain lines in Fig. 1).

Further, the X-ray detection unit 20 is provided with an AEC (Auto Exposure Control) 23. The AEC 23 is configured to receive X-rays irradiated by the X-ray irradiation unit 10. The AEC 23 is provided in the X-ray detection unit 20 and is a device that receives (detects) the X-rays after passing through the subject 101 and controls a tube voltage, a tube current, an irradiation time, and the like of an X-ray tube 11 (see Fig. 2) included in the X-ray irradiation unit 10 according to the received X-ray dose.

In X-ray imaging in the decubitus posture, the holding unit 40 is arranged at a position facing the X-ray detection unit 20 of the imaging table 21 in an up-down direction, and X-ray imaging is performed on the subject 101 lying on the imaging table 21 between the X-ray irradiation unit 10 and the X-ray detection unit 20 facing each other in the up-down direction, while the subject 101 lying on the imaging table 21 is imaged (optically imaged) by the optical imaging unit 31. In X-ray imaging in the upright posture, the holding unit 40 is arranged at a position facing the X-ray detection unit 20 of the imaging stand 22 in a horizontal direction, and X-ray imaging is performed on the subject 101 standing in front of the imaging stand 22 between the X-ray irradiation unit 10 and the X-ray detection unit 20 facing each other in the horizontal direction, while the subject 101 standing in front of the imaging stand 22 is imaged by the optical imaging unit 31.

The moving mechanism 50 is configured to movably hold the holding unit 40 in the horizontal direction (X direction and Y direction) and the vertical direction (Z direction). The moving mechanism 50 includes a ceiling suspender 51 and a column unit 52. The moving mechanism 50 is supported by rails 53 provided on the ceiling of the imaging room 110. The ceiling suspender 51 is configured to be movable in the horizontal direction by the rails 53. The ceiling suspender 51 is configured to support the column unit 52. The column unit 52 is configured to support the holding unit 40. The column unit 52 is configured to be extendable and retractable in the vertical direction. The holding unit 40 is configured to be movable in the vertical direction by the column unit 52. Moreover, the moving mechanism 50 moves the X-ray detection unit 20 arranged in each of the imaging table 21 and the imaging stand 22.

As shown in Fig. 2, the X-ray irradiation unit 10 includes the X-ray tube 11 and a collimator unit 12. The X-ray irradiation unit 10 is configured to irradiate X-rays from the X-ray tube 11 to the subject 101 (see Fig. 1). The X-ray tube 11 is configured to irradiate X-rays by a predetermined voltage being applied thereto. The collimator unit 12 has a plurality of position-adjustable shielding plates (collimator leaves). The collimator unit 12 is configured to adjust an irradiation field of the X-rays irradiated from the X-ray tube 11 by shielding a part of the X-rays from the X-ray tube 11. The collimator unit 12 is provided in the vicinity of the X-ray tube 11 in an X-ray irradiation direction of the X-ray tube 11.

The holding unit 40 includes a display operation unit 41 and a grip unit 42. The holding unit 40 is configured to be movable in the horizontal direction and the vertical direction via the moving mechanism 50 (see Fig. 1) by manual movement or control by the apparatus control unit 60 (see Fig. 1). In the first embodiment, the optical imaging unit 31 is provided on the holding unit 40 together with the X-ray irradiation unit 10. Specifically, an imaging unit 30 configured by the optical imaging unit 31 and an optical imaging control unit 32 is arranged on the holding unit 40. Moreover, in the first embodiment, a light source unit 33 and an input receiving unit 43 are also provided on the holding unit 40. Note that the optical imaging control unit 32 and the display operation unit 41 are examples of a "control unit" and a "display unit" in the claims, respectively.

The display operation unit 41 includes, for example, a touch panel type liquid crystal display. The display operation unit 41 functions as an image display unit that displays the optical image 70 (see Fig. 4) imaged by the optical imaging unit 31, and is configured to function as an operation unit into which various operations are input by an operator (such as a doctor and a technician). The display operation unit 41 outputs a signal indicating the received input operation to the optical imaging control unit 32 and the apparatus control unit 60.

The grip unit 42 is provided for the operator to grip when performing an operation to manually move the holding unit 40. The grip unit 42 transmits an operating force of the operator to the holding unit 40.

The optical imaging unit 31 is provided on an outer side surface of the collimator unit 12. In the first embodiment, the optical imaging unit 31 is provided on the outer side surface of the collimator unit 12 on a side facing a longitudinal direction of the imaging table 21 at the imaging position in the decubitus posture. Furthermore, the optical imaging unit 31 is provided on the outer side surface of the collimator unit 12 on a side surface side intersecting a detection surface of the X-ray detection unit 20 at the imaging position in the upright posture. The optical imaging unit 31 is provided facing the irradiation direction of the X-rays from the X-ray irradiation unit 10. The optical imaging unit 31 can capture the optical image 70 of the subject 101 and the X-ray detection unit 20 from the X-ray irradiation unit 10 side when the X-ray irradiation unit 10 faces the direction of the subject 101 and the X-ray detection unit 20. An imaging range of the optical imaging unit 31 is set so as to include a range of the irradiation field to which the X-rays are irradiated and to be larger than the range of the irradiation field of the X-rays.

Further, in the first embodiment, as shown in Fig. 2, the collimator unit 12 is provided with the light source unit 33 and the input receiving unit 43. The light source unit 33 is provided inside the collimator unit 12. The light source unit 33 is configured to irradiate visible light to an irradiation field region 33a (see Fig. 4) indicating a region to which the X-rays are irradiated by the X-ray irradiation unit 10. Specifically, a part of the visible light irradiated from the light source unit 33 is shielded by the plurality of shielding plates (collimator leaves) of the collimator unit 12 in the same manner as the X-rays irradiated from the X-ray irradiation unit 10, whereby the irradiation field is adjusted. Therefore, the irradiation field of the visible light irradiated from the light source unit 33 is adjusted by the plurality of shielding plates (collimator leaves) of the collimator unit 12, thereby becoming equal to the irradiation field of the X-rays irradiated from the X-ray irradiation unit 10. The light source unit 33 includes a light-emitting element such as an LED (Light Emitting Diode), for example.

Further, in the first embodiment, the input receiving unit 43 is configured to receive an input from the operator. The input receiving unit 43 is, for example, a push button for operating the light source unit 33. That is, the input receiving unit 43 is configured to receive an operation input to turn on the light source unit 33. The visible light is irradiated from the light source unit 33 by the operator operating (pressing) the input receiving unit 43.

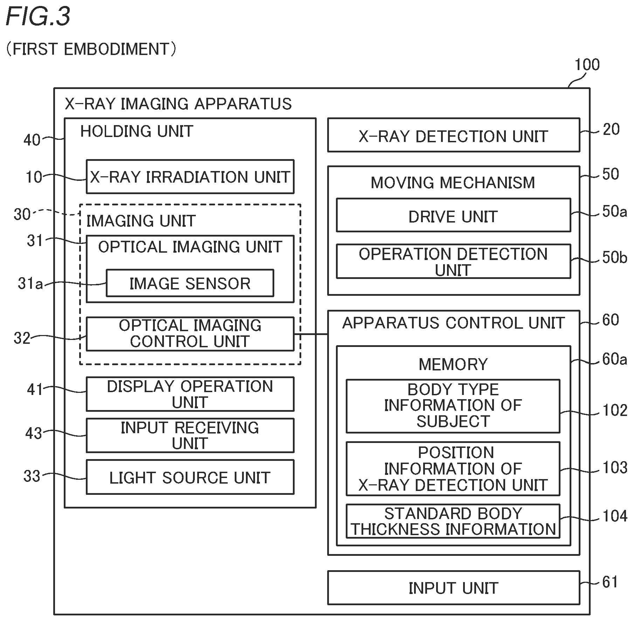

As shown in Fig. 3, the optical imaging unit 31 includes an image sensor 31a. The optical imaging unit 31 is, for example, an optical camera. The image sensor 31a includes, for example, a CCD (Charge Coupled Device) image sensor or a CMOS (Complementary Metal Oxide Semiconductor) image sensor. Further, the optical imaging control unit 32 is a microcomputer including, for example, a CPU (Central Processing Unit) and a memory. The optical imaging control unit 32 transmits and receives signals to and from the apparatus control unit 60 via a wireless connection or a wired connection.

As shown in Fig. 4, the optical imaging unit 31 (see Fig. 3) is configured to capture the optical image 70 of the subject 101. The optical imaging control unit 32 (see Fig. 3) is configured such that a signal output by the image sensor 31a (see Fig. 3) is input thereto, and generates the optical image 70 based on the input signal. Moreover, the optical imaging control unit 32 is configured to perform control to output the generated optical image 70 to the display operation unit 41 (see Fig. 3) and display it. Note that the example shown in Fig. 4 is the optical image 70 in a state where the visible light irradiated from the light source unit 33 (see Fig. 3) is irradiated to the irradiation field region 33a. Therefore, the optical image 70 shown in Fig. 4 shows the subject 101, the X-ray detection unit 20, and the irradiation field region 33a. Note that when the visible light is not irradiated from the light source unit 33, the irradiation field region 33a does not appear in the optical image 70.

Further, in the first embodiment, the optical imaging control unit 32 is configured to perform control to switch a display mode when displaying the optical image 70 from a first mode to a second mode. Details of the first mode and the second mode, and details of the configuration in which the optical imaging control unit 32 switches between the first mode and the second mode will be described later.

As shown in Fig. 3, the apparatus control unit 60 includes the memory 60a. The apparatus control unit 60 controls X-ray imaging by the X-ray irradiation unit 10 and the X-ray detection unit 20, and controls movement of the holding unit 40. Specifically, the apparatus control unit 60 includes a CPU. The input unit 61 has a function of receiving an input operation relating to X-ray imaging. The input operation is setting of imaging conditions for X-ray imaging, an instruction to start X-ray irradiation, or the like. The apparatus control unit 60 controls the X-ray imaging based on parameters and various programs preset and stored in the memory 60a. The apparatus control unit 60 controls an adjustment amount of the irradiation field by the collimator unit 12 (see Fig. 2).

In the first embodiment, the memory 60a stores body type information 102 of the subject 101, position information 103 of the X-ray detection unit 20, and standard body thickness information 104.

The body type information 102 of the subject 101 includes, for example, a height and a weight of the subject 101. The body type information 102 of the subject 101 is acquired from, for example, an imaging order of the subject 101, and stored in the memory 60a.

Further, the position information 103 of the X-ray detection unit 20 includes, for example, a movement amount of the X-ray detection unit 20 from an initial position, or position coordinates of the X-ray detection unit 20 after movement. Every time the X-ray detection unit 20 is moved, the position information 103 of the X-ray detection unit 20 is stored in the memory 60a.

Further, the standard body thickness information 104 is body thickness information preset for each imaging region of the subject 101. Specifically, the standard body thickness information 104 is a statistical value (average value) of a body thickness for each imaging region in a subject having a standard physical constitution. The standard body thickness information 104 is published, and is acquired in advance and stored in the memory 60a.

In the X-ray imaging apparatus 100, the moving mechanism 50 has a drive unit 50a such as a motor, and an operation detection unit 50b that detects the operation of the drive unit 50a. The operation detection unit 50b includes, for example, a potentiometer that detects rotation of the motor. The drive unit 50a and the operation detection unit 50b are arranged in each part of the moving mechanism 50 for moving the holding unit 40, and are arranged in each of the imaging table 21 (see Fig. 1) and the imaging stand 22 (see Fig. 1) to change the position of the X-ray detection unit 20. The apparatus control unit 60 changes a position and an angle of the X-ray irradiation unit 10 and a position of the X-ray detection unit 20 by controlling the operation of the moving mechanism 50 based on an input operation to the input unit 61 or the display operation unit 41, or an operating force applied to the grip unit 42 of the holding unit 40. Further, the apparatus control unit 60 controls the operation of the moving mechanism 50 by feedback control by outputting a control signal for controlling the operation of the drive unit 50a and receiving an input of a detection signal indicating the operation of the drive unit 50a detected by the operation detection unit 50b.

Then, in the first embodiment, the apparatus control unit 60 outputs the position and the angle of the X-ray irradiation unit 10 held by the holding unit 40, and the position of the X-ray detection unit 20 arranged in each of the imaging table 21 and the imaging stand 22 to the optical imaging control unit 32. The optical imaging control unit 32 acquires the arrangement of the X-ray irradiation unit 10 and the arrangement of the X-ray detection unit 20 based on the input from the apparatus control unit 60. Moreover, the optical imaging control unit 32 acquires the adjustment amount of the irradiation field by the collimator unit 12 based on the input from the apparatus control unit 60. Further, in the first embodiment, the apparatus control unit 60 stores the position information 103 of the X-ray detection unit 20 in the memory 60a.

### (Display Control by Optical Imaging Control Unit)

As shown in Fig. 5, in the first embodiment, the optical imaging control unit 32 (see Fig. 3) is configured to superimpose a region display 80 on the optical image 70 and to perform control to display it on the display operation unit 41 (see Fig. 3). Note that in the following description, the display control of the display operation unit 41 by the optical imaging control unit 32 when performing X-ray imaging in a posture in which the subject 101 stands up (upright posture) will be described. Further, since the above control when performing X-ray imaging in a posture in which the subject 101 is laid on the imaging table 21 (see Fig. 1) (decubitus posture) is similar control, description thereof is omitted. Moreover, Figs. 5 to 7 show an example in the case of performing X-ray imaging of a chest of the subject 101.

The region display 80 includes at least any one of a detection unit region display 81 indicating a region where the X-ray detection unit 20 is arranged in the optical image 70, an X-ray irradiation field region display 84 indicating a region to which X-rays are irradiated by the X-ray irradiation unit 10 (irradiation field region 33a (see Fig. 4)) in the optical image 70, and an AEC region display 87 indicating a region that receives X-rays irradiated by the X-ray irradiation unit 10 in the optical image 70. In the example shown in Fig. 5, the region display 80 includes all of the detection unit region display 81, the X-ray irradiation field region display 84, and the AEC region display 87. Note that the AEC region display 87 is an example of an "X-ray light receiving field region display" in the claims.

The detection unit region display 81 indicates a region where the X-ray detection unit 20 is arranged in the optical image 70. The optical imaging control unit 32 acquires a three-dimensional arrangement of the X-ray irradiation unit 10 (see Fig. 3) and the X-ray detection unit 20, and acquires a three-dimensional arrangement of the optical imaging unit 31 (see Fig. 3). Since the optical imaging unit 31 is arranged in the holding unit 40 (see Fig. 3) together with the X-ray irradiation unit 10, the optical imaging control unit 32 calculates a three-dimensional position of the optical imaging unit 31 using parameters stored in advance, based on the acquired position of the X-ray irradiation unit 10, for example. Then, the optical imaging control unit 32 detects a region where the X-ray detection unit 20 is arranged in the optical image 70 by performing geometrical calculations based on the acquired three-dimensional positional relationship among the X-ray irradiation unit 10, the X-ray detection unit 20, and the optical imaging unit 31. Note that the optical imaging control unit 32 may acquire the position of the optical imaging unit 31 calculated by the apparatus control unit 60.

The optical imaging control unit 32 generates the detection unit region display 81 of the region display 80 based on the detected region where the X-ray detection unit 20 is arranged. The detection unit region display 81 indicates the region where the X-ray detection unit 20 is arranged in a rectangular shape. The detection unit region display 81 indicates, for example, a square region. The detection unit region display 81 has a center display 82 indicating the center of the region where the X-ray detection unit 20 is arranged, and four region displays 83 indicating the four corners of the rectangular region by L-shapes. The center display 82 has a cross shape, and is arranged at a position where diagonal lines of the rectangular region intersect. The center display 82 and the four region displays 83 are illustrated in white for convenience, but are displayed by being colored in blue, for example. Further, the detection unit region display 81 shows a guide for the position and range of a region capable of detecting X-rays in the X-ray detection unit 20 in the optical image 70. Note that the region indicated by the detection unit region display 81 may be a smaller range or a larger range than the region actually capable of detecting X-rays in the X-ray detection unit 20.

Further, as shown in Fig. 5, the optical imaging control unit 32 is configured to superimpose the X-ray irradiation field region display 84 and the AEC region display 87 as the region display 80 on the optical image 70 and perform control to display them on the display operation unit 41. Similar to the detection unit region display 81, the optical imaging control unit 32 detects the irradiation field region 33a to which X-rays are irradiated by the X-ray irradiation unit 10 in the optical image 70 by performing geometrical calculations based on the acquired three-dimensional positional relationship among the X-ray irradiation unit 10, the X-ray detection unit 20, and the optical imaging unit 31. Then, the optical imaging control unit 32 generates the X-ray irradiation field region display 84 based on the detected irradiation field region 33a to which X-rays are irradiated.

The X-ray irradiation field region display 84 indicates the region to which X-rays are irradiated in a rectangular shape, similar to the detection unit region display 81. The X-ray irradiation field region display 84 indicates, for example, a square region. The X-ray irradiation field region display 84 has a region display 85 which is a rectangular frame line indicating the irradiation field region 33a to which X-rays are irradiated, and a pair of straight dotted line displays 86 orthogonal to each other so as to indicate the center of the irradiation field region 33a to which X-rays are irradiated. The pair of dotted line displays 86 are arranged while being orthogonal to each other so as to pass through a center position where diagonal lines of the rectangle of the region display 85 intersect. Further, the pair of dotted line displays 86 are arranged so as to extend across the outside of the region display 85 which is the rectangular frame line. The region display 85 and the pair of dotted line displays 86 are illustrated in white for convenience, but are displayed by being colored in a color different from the detection unit region display 81 (for example, yellow). Further, the X-ray irradiation field region display 84 shows a guide for the position and range of the region to which X-rays are irradiated in the optical image 70. Note that the region indicated by the X-ray irradiation field region display 84 may be a smaller range or a larger range than the region to which X-rays are actually irradiated.

In the first embodiment, the optical imaging control unit 32 is configured to change the size of the X-ray irradiation field region display 84 superimposed on the optical image 70 in the display operation unit 41, according to the irradiation field of X-rays defined by the adjustment of the collimator unit 12. The optical imaging control unit 32 changes the size of the region display 85 which is the rectangular frame line in the X-ray irradiation field region display 84 superimposed on the optical image 70, based on a value of the adjustment amount of the collimator unit 12 acquired from the apparatus control unit 60. Note that even if the size of the region display 85 indicated by the rectangular frame line is changed in the X-ray irradiation field region display 84, the size of the dotted line displays 86 is not changed.

The AEC region display 87 indicates the position and shape of the AEC 23 (see Fig. 1) provided in the X-ray detection unit 20. The position and shape where the AEC 23 is provided in the X-ray detection unit 20 are known. Moreover, as described above, when generating the detection unit region display 81, the optical imaging control unit 32 detects the region where the X-ray detection unit 20 is arranged in the optical image 70. Therefore, the optical imaging control unit 32 generates the AEC region display 87 based on the detected arrangement of the X-ray detection unit 20 and the position and shape of the AEC 23 in the X-ray detection unit 20. The AEC is provided in the X-ray detection unit 20. Therefore, the AEC region display 87 is illustrated in white for convenience, but is displayed by being colored in the same color as the detection unit region display 81 (for example, blue). In the example shown in Fig. 5, two AEC region displays 87 are superimposed on the optical image 70.

### (Display of Optical Image by First Mode)

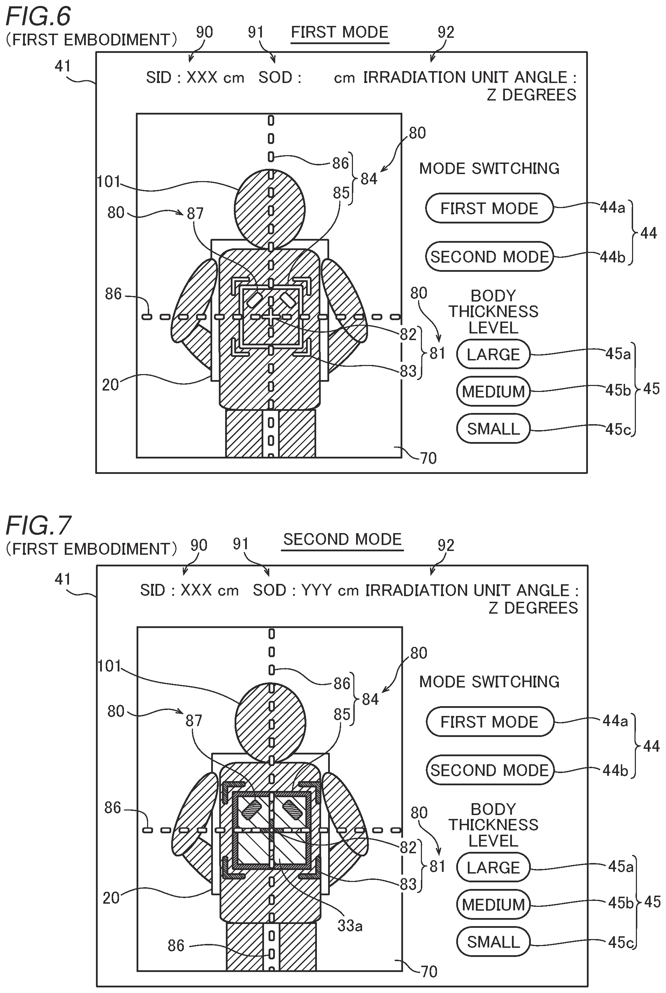

Next, a configuration in which the optical imaging control unit 32 (see Fig. 3) displays the optical image 70 by the first mode will be described with reference to Fig. 6.

In the first embodiment, in the first mode, the optical imaging control unit 32 indicates the region where the X-ray detection unit 20 is arranged in the optical image 70, and displays the optical image 70 superimposed with the region display 80 based on a predetermined fixed position in a real space, or displays the optical image 70 with the region display 80 hidden, without superimposing the region display 80 on the optical image 70. Fig. 6 illustrates an example in which the optical imaging control unit 32 displays the optical image 70 superimposed with the region display 80 including the detection unit region display 81, the X-ray irradiation field region display 84, and the AEC region display 87 based on the fixed position. The first mode is a mode for displaying the optical image 70 superimposed with the region display 80 based on the fixed position on the display operation unit 41 when position adjustment of the subject 101 is performed.

In the first embodiment, when superimposing the region display 80 on the optical image 70 to display it in the first mode, the optical imaging control unit 32 is configured to perform control to superimpose the region display 80 including the detection unit region display 81, the X-ray irradiation field region display 84, and the AEC region display 87 based on the fixed position on the optical image 70 to display it on the display operation unit 41, based on the position information 103 (see Fig. 3) of the X-ray detection unit 20 or the standard body thickness information 104 (see Fig. 3).

When the optical imaging control unit 32 superimposes the region display 80 including the detection unit region display 81, the X-ray irradiation field region display 84, and the AEC region display 87 on the optical image 70 based on the fixed position based on the position information 103 of the X-ray detection unit 20, the detection unit region display 81, the X-ray irradiation field region display 84, and the AEC region display 87 are superimposed on a surface of the X-ray detection unit 20 facing the X-ray irradiation unit 10 (see Fig. 3) (the front surface of the X-ray detection unit 20). Moreover, when the optical imaging control unit 32 superimposes the region display 80 including the detection unit region display 81, the X-ray irradiation field region display 84, and the AEC region display 87 on the optical image 70 based on the fixed position based on the standard body thickness information 104, the detection unit region display 81, the X-ray irradiation field region display 84, and the AEC region display 87 are superimposed at a position on the X-ray irradiation unit 10 side by an amount of a body thickness acquired based on the standard body thickness information 104 from the front surface of the X-ray detection unit 20. Fig. 6 shows an example in which the optical imaging control unit 32 displays the optical image 70 superimposed with the region display 80 including the detection unit region display 81, the X-ray irradiation field region display 84, and the AEC region display 87 based on the fixed position based on the position information 103 of the X-ray detection unit 20.

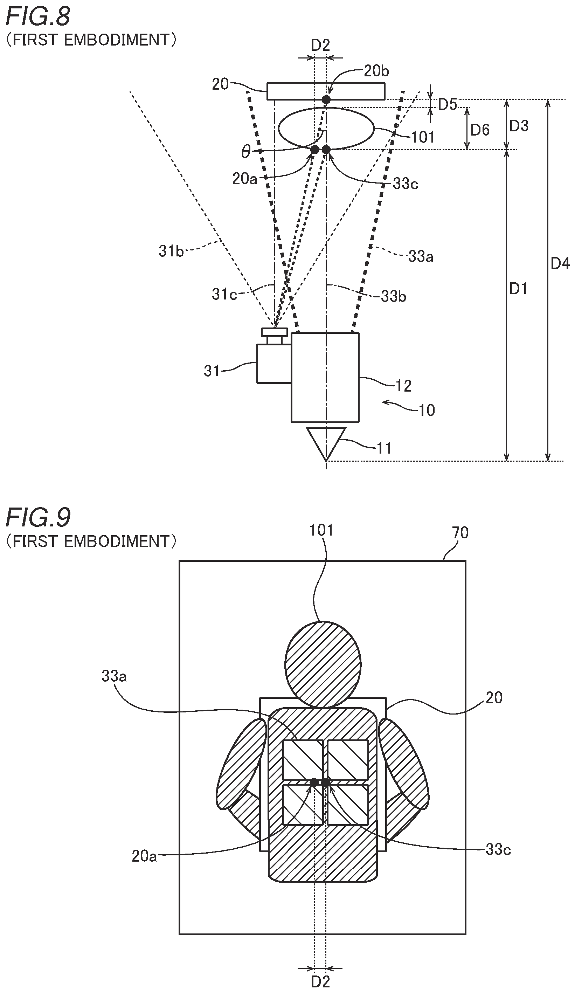

Further, as shown in Fig. 6, in the first embodiment, the optical imaging control unit 32 causes the display operation unit 41 to display an SID display region 90 that displays an SID (Source to Image receptor Distance) which is a distance between the X-ray irradiation unit 10 and the X-ray detection unit 20 (X-ray tube-detector distance D4 (see Fig. 8)). Moreover, the optical imaging control unit 32 causes the display operation unit 41 to display an SOD display region 91 that displays an SOD (Source to Object Distance) which is a distance between the X-ray irradiation unit 10 and a body surface 101a (see Fig. 1) of the subject 101 (X-ray tube-subject distance D1 (see Fig. 8)). In the example shown in Fig. 6, since the SOD is not acquired, a value of the SOD is not displayed in the SOD display region 91. Note that in this specification, the body surface 101a of the subject 101 means a front surface of the subject 101 on the X-ray irradiation unit 10 side when the subject 101 is arranged between the X-ray irradiation unit 10 and the X-ray detection unit 20. Further, the optical imaging control unit 32 causes the display operation unit 41 to display an irradiation unit angle display region 92 that displays an angle of the X-ray irradiation unit 10 with respect to the imaging table 21 (see Fig. 1) or the imaging stand 22 (see Fig. 1).

### (Display of Optical Image by Second Mode)

Next, a configuration in which the optical imaging control unit 32 (see Fig. 3) displays the optical image 70 by the second mode will be described with reference to Fig. 7.

The optical imaging control unit 32 displays the optical image 70 superimposed with the region display 80 based on a position on the body surface 101a (see Fig. 1) of the subject 101 in the real space in the second mode. In the first embodiment, when displaying the optical image 70 in the second mode, the optical imaging control unit 32 is configured to perform control to superimpose the region display 80 including the detection unit region display 81, the X-ray irradiation field region display 84, and the AEC region display 87 on the optical image 70 and to display it on the display operation unit 41. Note that the second mode is a mode for displaying the optical image 70 superimposed with the region display 80 on the body surface 101a of the subject 101 on the display operation unit 41 when position adjustment of the X-ray irradiation unit 10 is performed. In the first embodiment, when displaying the optical image 70 in the second mode, the optical imaging control unit 32 is configured to perform control to display the optical image 70 superimposed with the region display 80 deformed according to the body thickness of the subject 101, based on the position on the body surface 101a of the subject 101 in the real space, on the display operation unit 41.

### (Switching between First Mode and Second Mode)

The optical imaging control unit 32 is configured to perform control to switch from the first mode to the second mode based on an input from the operator received by the input receiving unit 43 (see Fig. 3) when position adjustment is performed. In the first embodiment, the optical imaging control unit 32 is configured to perform control to switch a mode for displaying the optical image 70 from the first mode to the second mode based on receiving an operation input to turn on the light source unit 33 (see Fig. 3).

As shown in Figs. 6 and 7, the optical imaging control unit 32 is configured to perform control to cause a display aspect of the region display 80 (the detection unit region display 81, the X-ray irradiation field region display 84, and the AEC region display 87) in the first mode to differ from a display aspect of the region display 80 (the detection unit region display 81, the X-ray irradiation field region display 84, and the AEC region display 87) in the second mode, to superimpose it on the optical image 70, and to display it on the display operation unit 41. The optical imaging control unit 32 makes the display aspect of the region display 80 differ by changing a shape and/or a color of the region display 80 (the detection unit region display 81, the X-ray irradiation field region display 84, and the AEC region display 87), for example. In Figs. 6 and 7, by varying the hatching of the detection unit region display 81, the X-ray irradiation field region display 84, and the AEC region display 87, differences in color of the detection unit region display 81, the X-ray irradiation field region display 84, and the AEC region display 87 are illustrated. Note that in Figs. 6 and 7, although the display aspect of the pair of dotted line displays 86 in the X-ray irradiation field region display 84 is not differentiated between the first mode and the second mode, the display aspect of the pair of dotted line displays 86 may also be differentiated between the first mode and the second mode, along with the region display 85 which is the rectangular frame line. Moreover, in the first embodiment, since the optical imaging control unit 32 switches between the first mode and the second mode based on turning on the light source unit 33, the irradiation field region 33a does not appear in the optical image 70 in the first mode shown in Fig. 6, but the irradiation field region 33a appears in the optical image 70 in the second mode shown in Fig. 7.

Further, in the first embodiment, since the fixed position in the first mode is the front surface of the X-ray detection unit 20, the size of the region display 80 (the detection unit region display 81, the X-ray irradiation field region display 84, and the AEC region display 87) superimposed on the optical image 70 shown in Fig. 6 is smaller than the size of the region display 80 (the detection unit region display 81, the X-ray irradiation field region display 84, and the AEC region display 87) superimposed on the optical image 70 shown in Fig. 7.

In the first mode, the detection unit region display 81, the X-ray irradiation field region display 84, and the AEC region display 87 are displayed based on a predetermined fixed position in the real space preset in the irradiation direction of X-rays in a state where the X-ray irradiation unit 10 and the X-ray detection unit 20 face each other, regardless of the position of the body surface 101a (see Fig. 1) of the subject 101. Therefore, when superimposing the detection unit region display 81, the X-ray irradiation field region display 84, and the AEC region display 87 in the first mode, the optical imaging control unit 32 superimposes them with their colors made lighter, for example. Further, in the second mode, the detection unit region display 81, the X-ray irradiation field region display 84, and the AEC region display 87 are superimposed on the body surface 101a of the subject 101. Therefore, in the second mode, the optical imaging control unit 32 superimposes the detection unit region display 81, the X-ray irradiation field region display 84, and the AEC region display 87 with their colors made darker. That is, the optical imaging control unit 32 varies the display aspect of the region display 80 (the detection unit region display 81, the X-ray irradiation field region display 84, and the AEC region display 87) based on a difference in accuracy of the position and shape in the first mode and the second mode.

Note that in the first embodiment, as shown in Figs. 6 and 7, the optical imaging control unit 32 may be configured to switch between the first mode and the second mode based on a mode switching button 44 displayed on the display operation unit 41. The mode switching button 44 is a GUI (Graphical User Interface) button displayed on the display operation unit 41. The mode switching button 44 includes a first mode switching button 44a and a second mode switching button 44b. When the first mode switching button 44a is pressed, the optical imaging control unit 32 displays the optical image 70 by the first mode. Further, when the second mode switching button 44b is pressed, the optical imaging control unit 32 displays the optical image 70 by the second mode. Accordingly, the operator can switch between the first mode and the second mode at any timing. Therefore, convenience (usability) for the operator can be improved.

### (Control of Superimposed Image based on Body Thickness of Subject)

The optical imaging control unit 32 is configured to acquire the body thickness of the subject 101 based on any one of the optical image 70 captured by the optical imaging unit 31, an operator's selection operation of body thickness information of the subject 101, and the body type information 102 (see Fig. 3) of the subject 101. Further, the optical imaging control unit 32 is configured to perform control to display the optical image 70 superimposed with the region display 80 deformed according to the acquired body thickness of the subject 101, based on the position on the body surface 101a of the subject 101 in the real space, on the display operation unit 41 in the second mode.

### (Calculation of Body Thickness of Subject)

Next, a configuration in which the optical imaging control unit 32 calculates the body thickness of the subject 101 will be described with reference to Figs. 8 and 9.

As shown in Figs. 8 and 9, the optical imaging control unit 32 (see Fig. 3) calculates the X-ray tube-subject distance D1 between the X-ray tube 11 (focal point thereof) and the subject 101, based on coordinates of a center as a predetermined position 33c in the irradiation field region 33a appearing on the body surface of the subject 101 in the optical image 70 captured by the optical imaging unit 31 (see Fig. 3).

Specifically, the optical imaging control unit 32 (see Fig. 3) calculates the detector-body surface distance D3 between the X-ray detection unit 20 and the body surface of the subject 101 in the optical axis direction of the light source unit 33 (see Fig. 3), based on a coordinate difference D2 between coordinates of a center as a predetermined position 20a of the X-ray detection unit 20 in the optical image 70 and coordinates of the center as the predetermined position 33c in the irradiation field region 33a appearing on the body surface of the subject 101 in the optical image 70. Then, the optical imaging control unit 32 calculates the X-ray tube-subject distance D1 based on the calculated detector-body surface distance D3 and the X-ray tube-detector distance D4 between the X-ray tube 11 and the X-ray detection unit 20. Note that the X-ray tube-detector distance D4 is calculated by the optical imaging control unit 32 based on the position of the X-ray irradiation unit 10 and the position of the X-ray detection unit 20 acquired by the operation detection unit 50b (see Fig. 3).

As shown in Fig. 8, an optical axis 31c of the optical imaging unit 31 is parallel to the optical axis 33b of the light source unit 33. In other words, the optical imaging unit 31 has an optical axis shifted in a direction in which the optical imaging unit 31 and the X-ray irradiation unit 10 are aligned, with respect to an irradiation axis of X-rays irradiated from the X-ray irradiation unit 10 (the optical axis 33b of the light source unit 33). Therefore, as shown in Fig. 9, in the optical image 70, the center as the predetermined position 20a of the X-ray detection unit 20 and the center as the predetermined position 33c in the irradiation field region 33a appearing on the body surface of the subject 101 are shifted from each other. Therefore, the optical imaging control unit 32 (see Fig. 3) first calculates the coordinate difference D2 between the coordinates of the center as the predetermined position 20a of the X-ray detection unit 20 in the optical image 70 and the coordinates of the center as the predetermined position 33c in the irradiation field region 33a appearing on the body surface of the subject 101 in the optical image 70. Note that in Fig. 8, an imaging range 31b of the optical imaging unit 31 is illustrated by dashed lines. The imaging range 31b of the optical imaging unit 31 is set so as to include the range of the irradiation field to which the X-rays are irradiated and to be larger than the range of the irradiation field of the X-rays.

Next, as shown in Fig. 8, the optical imaging control unit 32 (see Fig. 3) calculates the detector-body surface distance D3 between the X-ray tube 11 and the X-ray detection unit 20 from the equation tan θ = (coordinate difference D2 / detector-body surface distance D3), where θ is the angle formed by the optical axis 33b of the light source unit 33 and the straight line connecting the optical imaging unit 31 and the center 20b of the X-ray detector 20. That is, the optical imaging control unit 32 calculates the detector-body surface distance D3 in the optical axis direction of the X-ray tube 11 based on the angle θ formed by the optical axis 33b of the light source unit 33 and the straight line connecting the optical imaging unit 31 and the center 20b of the X-ray detector 20, in addition to the coordinate difference D2. The body thickness of the subject 101 is the distance D6 obtained by subtracting the detector-body back surface distance D5 from the X-ray detector 20 to the subject 101 (the back surface thereof) in the optical axis direction of the light source unit 33 from the detector-body surface distance D3. Further, the detector-body back surface distance D5 is calculated based on an arrangement of the X-ray detection unit 20 held on the imaging table 21 and a thickness of the imaging table 21 in the optical axis direction of the light source unit 33. The arrangement of the X-ray detection unit 20 held on the imaging table 21 and the thickness of the imaging table 21 in the optical axis direction of the light source unit 33 are known values. Therefore, the optical imaging control unit 32 calculates the detector-body back surface distance D5 from the arrangement of the X-ray detection unit 20 and the thickness of the imaging table 21 in the optical axis direction of the light source unit 33, which are known values. Then, the optical imaging control unit 32 acquires the distance D6 calculated by subtracting the detector-body back surface distance D5 from the detector-body surface distance D3 as the body thickness of the subject 101.

Further, in the first embodiment, the optical imaging control unit 32 calculates the X-ray tube-subject distance D1 by subtracting the detector-body surface distance D3 from the X-ray tube-detector distance D4. Further, as shown in Fig. 7, the optical imaging control unit 32 displays the calculated X-ray tube-subject distance D1 in the SOD display region 91. Furthermore, the optical imaging control unit 32 displays the X-ray tube-detector distance D4 in the SID display region 90.

### (Acquisition of Body Thickness by Selection Operation of Body Thickness Information)

As shown in Figs. 6 and 7, the optical imaging control unit 32 (see Fig. 3) may be configured to acquire the body thickness of the subject 101 based on an operation input to a body thickness level selection button 45 by the operator. The body thickness level selection button 45 is a GUI button displayed on the display operation unit 41. The body thickness level selection button 45 includes a "large" button 45a, a "medium" button 45b, and a "small" button 45c. The optical imaging control unit 32 acquires a predetermined value according to the "large" button 45a, the "medium" button 45b, and the "small" button 45c as the body thickness of the subject 101. Then, when displaying the optical image 70 in the second mode, the optical imaging control unit 32 displays the region display 80 on the body surface 101a of the subject 101 based on the body thickness according to the selected body thickness level selection button 45.

For example, assuming that values corresponding to the "large" button 45a, the "medium" button 45b, and the "small" button 45c are respectively stored as "60 cm", "40 cm", and "20 cm" in the memory 60a (see Fig. 3), when the "medium" button 45b is pressed, the optical imaging control unit 32 sets a position away from the front surface of the X-ray detection unit 20 toward the X-ray irradiation unit 10 side by 40 cm as the body surface 101a of the subject 101. Then, the optical imaging control unit 32 displays the optical image 70 on which the region display 80 is superimposed at a position away from the front surface of the X-ray detection unit 20 toward the X-ray irradiation unit 10 side by 40 cm, on the display operation unit 41. Note that the above values corresponding to the "large" button 45a, the "medium" button 45b, and the "small" button 45c are examples, and may be set to different values.

### (Calculation of Body Thickness based on Body Type Information of Subject)

The optical imaging control unit 32 may acquire the body thickness of the subject 101 based on the body type information 102 (see Fig. 3) of the subject 101. Specifically, the optical imaging control unit 32 calculates the body thickness of the subject 101 from a height and a weight of the subject 101 included in the body type information 102 by a known calculation formula. Then, when displaying the optical image 70 in the second mode, the optical imaging control unit 32 displays the region display 80 and the like on the body surface 101a of the subject 101 based on the body thickness calculated based on the body type information 102 of the subject 101.

### (Image Display Method)

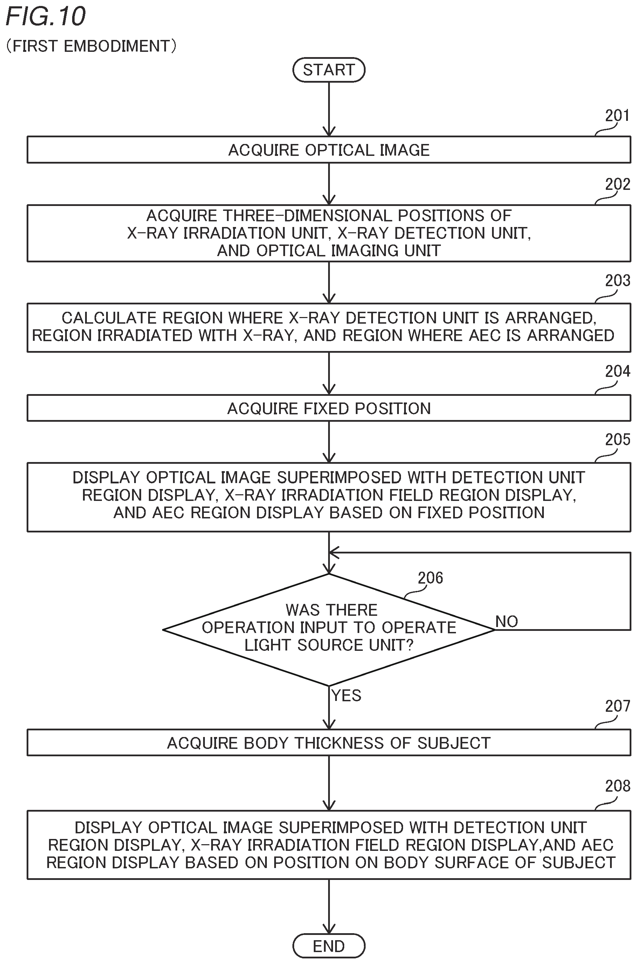

Next, referring to Fig. 10, control processing of an image display method by the X-ray imaging apparatus 100 (see Fig. 1) of the first embodiment will be described. The control processing of the image display method by steps 201 to 208 is executed by the optical imaging control unit 32 (see Fig. 3).

First, in step 201, the optical image 70 (see Fig. 4) captured by the optical imaging unit 31 (see Fig. 3) is acquired.

Next, in step 202, based on a signal from the apparatus control unit 60 (see Fig. 3), the three-dimensional positions of the X-ray irradiation unit 10 (see Fig. 3) and the X-ray detection unit 20 (see Fig. 3) are acquired, and the three-dimensional position of the optical imaging unit 31 is calculated and acquired. Further, based on the three-dimensional position of the X-ray detection unit 20, a three-dimensional position of the AEC 23 (see Fig. 1) is also calculated and acquired.

Next, in step 203, a region where the X-ray detection unit 20 is arranged, a region to which X-rays are irradiated (irradiation field region 33a (see Fig. 4)), and a region where the AEC 23 is arranged in the optical image 70 are calculated.

Next, in step 204, a fixed position is acquired. In the first embodiment, the fixed position may be acquired based on the position information 103 (see Fig. 3) of the X-ray detection unit 20, or may be acquired based on the standard body thickness information 104.

Next, in step 205, as shown in Fig. 6, the optical image 70 superimposed with the region display 80 (the detection unit region display 81, the X-ray irradiation field region display 84, and the AEC region display 87) based on a predetermined fixed position in the real space is displayed on the display operation unit 41.

Next, in step 206, it is determined whether or not there has been an operation input to operate the light source unit 33. If there is an operation input to operate the light source unit 33, the processing proceeds to step 207. If there is no operation input to operate the light source unit 33, the processing of step 206 is repeated.

Next, in step 207, the optical imaging control unit 32 acquires the body thickness of the subject 101. The optical imaging control unit 32 acquires the body thickness of the subject 101 based on the optical image 70. Note that the optical imaging control unit 32 may acquire the body thickness of the subject 101 based on an operation input to the body thickness level selection button 45 (see Fig. 6). Further, the optical imaging control unit 32 may acquire the body thickness of the subject 101 based on the body type information 102 (see Fig. 3) of the subject 101.

Next, in step 208, the optical imaging control unit 32 displays the optical image 70 superimposed with the region display 80 (the detection unit region display 81, the X-ray irradiation field region display 84, and the AEC region display 87) based on the position on the body surface 101a of the subject 101 in the real space, on the display operation unit 41. Thereafter, the processing ends.

Note that either of step 201 or step 202 may be executed first in the control processing.

### (Effects of First Embodiment)

In the first embodiment, the following effects can be obtained.

In the first embodiment, as described above, the X-ray imaging apparatus 100 includes the X-ray irradiation unit 10 including the X-ray tube 11, the X-ray detection unit 20 configured to detect X-rays irradiated from the X-ray irradiation unit 10 and passed through the subject 101, the optical imaging unit 31 configured to capture the optical image 70 of the subject 101, the display operation unit 41 configured to display the optical image 70 captured by the optical imaging unit 31, the optical imaging control unit 32 configured to perform control to display the optical image 70 on the display operation unit 41, and the input receiving unit 43 configured to receive an input from the operator. The optical imaging control unit 32 is configured to perform control to switch from the first mode of displaying the optical image 70 superimposed with the region display 80 including at least one of the detection unit region display 81 indicating a region where the X-ray detection unit 20 is arranged in the optical image 70, the X-ray irradiation field region display 84 indicating a region to which X-rays are irradiated by the X-ray irradiation unit 10 in the optical image 70, and the AEC region display 87 indicating a region that receives X-rays irradiated by the X-ray irradiation unit 10 in the optical image 70, based on a predetermined fixed position in the real space, or displaying the optical image 70 with the region display 80 hidden, without superimposing the region display 80 on the optical image 70, to the second mode of displaying the optical image 70 superimposed with the region display 80 based on the position on the body surface 101a of the subject 101 in the real space, based on an input from the operator received by the input receiving unit 43 when performing position adjustment.

Thus, when the optical image 70 superimposed with the region display 80 is displayed in the first mode, the optical image 70 superimposed with the region display 80 at the fixed position is displayed. Therefore, even if the position of the body surface 101a of the subject 101 changes when a rough position adjustment between the subject 101 and the X-ray irradiation unit 10 is performed, the region display 80 can be displayed in a fixed manner. Accordingly, it is possible to prevent the position and shape of the region display 80 from changing, so that it is possible to suppress a decrease in work efficiency of position adjustment caused by frequent changes in the position and shape of the region display 80 displayed when performing rough position adjustment between the subject 101 and the X-ray irradiation unit 10. Further, when the optical image 70 is displayed with the region display 80 not superimposed and hidden in the first mode, the region display 80 is not superimposed on the optical image 70, so that it is possible to prevent a decrease in work efficiency of position adjustment caused by frequent changes in the position and shape of the region display 80 displayed when performing rough position adjustment between the subject 101 and the X-ray irradiation unit 10. Furthermore, after the rough position adjustment between the subject 101 and the X-ray irradiation unit 10, the operator performs fine adjustment of the positions of the subject 101 and the X-ray irradiation unit 10. In the fine adjustment of the positions of the subject 101 and the X-ray irradiation unit 10, position adjustment between the subject 101 and the X-ray irradiation unit 10 is performed so that the region of the X-rays irradiated to the body surface of the subject 101 becomes a position corresponding to a detection region of the X-ray detection unit 20. In the second mode, which is switched from the first mode based on an input from the operator, the optical image 70 is displayed with the region display 80 superimposed on the body surface of the subject 101. Therefore, the operator can easily perform position adjustment while visually recognizing the region display 80 superimposed on the body surface 101a of the subject 101 in the optical image 70 when performing fine adjustment of the positions of the subject 101 and the X-ray irradiation unit 10. As a result of these, it is possible to provide the X-ray imaging apparatus 100 capable of easily performing position adjustment while suppressing a decrease in work efficiency.

Moreover, in the first embodiment, the following further effects can be obtained by configuring as follows.

That is, in the first embodiment, as described above, the first mode is a mode for displaying the optical image 70 superimposed with the region display 80 based on the fixed position on the display operation unit 41 (display unit) when position adjustment of the subject 101 is performed, and the second mode is a mode for displaying the optical image 70 superimposed with the region display 80 based on the position on the body surface 101a of the subject 101 in the real space on the display operation unit 41 when position adjustment of the X-ray irradiation unit 10 is performed. Here, when position adjustment of the subject 101 is performed, the position adjustment of the subject 101 may be performed while visually recognizing the position of the X-ray detection unit 20 to serve as a guide for position adjustment. Therefore, by configuring as described above, when performing position adjustment of the subject 101, the optical image 70 superimposed with the region display 80 based on the fixed position is displayed by the first mode, so that the position adjustment of the subject 101 can be performed while visually recognizing the region display 80 as a guide for position adjustment. At that time, even if the position of the body surface 101a of the subject 101 changes, the region display 80 is fixedly displayed, so that it is possible to suppress a decrease in work efficiency of position adjustment of the subject 101 caused by frequent changes in the position and shape of the region display 80. Further, when position adjustment of the X-ray irradiation unit 10 is performed, the optical image 70 superimposed with the region display 80 based on the position on the body surface 101a of the subject 101 is displayed by the second mode, so that when performing position adjustment of the X-ray irradiation unit 10, the operator can easily perform position adjustment of the X-ray irradiation unit 10 by visually recognizing the region display 80 superimposed on the optical image 70.

Furthermore, in the first embodiment, as described above, the optical imaging control unit 32 (control unit) is configured to perform control to display the optical image 70 superimposed with the region display 80 deformed according to the body thickness of the subject 101 based on the position on the body surface 101a of the subject 101 in the real space, on the display operation unit 41 (display unit) when displaying the optical image 70 in the second mode.

Here, the region display 80 includes any one of the detection unit region display 81 indicating a region detected by the X-ray detection unit 20 among X-rays irradiated to the subject 101, the X-ray irradiation field region display 84 indicating a region of X-rays irradiated from the X-ray irradiation unit 10 (irradiation field region 33a), and the AEC region display 87 indicating a region that receives X-rays irradiated by the X-ray irradiation unit 10. Therefore, as described above, by displaying the optical image 70 superimposed with the region display 80 deformed according to the body thickness of the subject 101 based on the position on the body surface 101a of the subject 101 in the real space on the display operation unit 41, if the region display 80 includes the detection unit region display 81, the operator can easily confirm the region where the X-rays transmitted through the subject 101 are detected by the X-ray detection unit 20 by visually recognizing the optical image 70. As a result, by performing position adjustment of the X-ray irradiation unit 10 while visually recognizing the detection unit region display 81 included in the region display 80 displayed on the body surface 101a of the subject 101, the position adjustment of the X-ray irradiation unit 10 can be easily performed so as to be at an appropriate position according to the body thickness of the subject 101. Further, when the region display 80 includes the X-ray irradiation field region display 84, the operator can easily confirm the irradiation field region 33a of X-rays irradiated onto the body surface 101a of the subject 101 by visually recognizing the optical image 70. As a result, by performing position adjustment of the X-ray irradiation unit 10 while visually recognizing the X-ray irradiation field region display 84 included in the region display 80 displayed on the body surface 101a of the subject 101, position adjustment of the X-ray irradiation unit 10 can be easily performed so as to be at an appropriate position according to the body thickness of the subject 101. Further, when the region display 80 includes the AEC region display 87, the operator can easily confirm the region where the AEC 23 for receiving X-rays irradiated from the X-ray irradiation unit 10 is arranged by visually recognizing the optical image 70. As a result, by performing position adjustment of the X-ray irradiation unit 10 while visually recognizing the AEC region display 87 included in the region display 80 displayed on the body surface 101a of the subject 101, position adjustment of the X-ray irradiation unit 10 and the X-ray detection unit 20 can be easily performed so as to be at an appropriate position according to the body thickness of the subject 101.

Moreover, in the first embodiment, as described above, the light source unit 33 for irradiating visible light to the irradiation field region 33a indicating a region to which X-rays are irradiated by the X-ray irradiation unit 10 is further provided, the input receiving unit 43 is configured to receive an operation input to turn on the light source unit 33, and the optical imaging control unit 32 (control unit) is configured to perform control to switch the mode for displaying the optical image 70 from the first mode to the second mode based on receiving the operation input to turn on the light source unit 33. Here, when performing position adjustment of the subject 101 and the X-ray irradiation unit 10, after rough position adjustment of the subject 101 is performed, position adjustment (fine adjustment) of the X-ray irradiation unit 10 is performed. At this time, adjustment of the irradiation field of X-rays by the collimator unit 12 is also performed together. When adjusting the irradiation field of the collimator unit 12, it is performed by operating the collimator unit 12 while irradiating visible light from the light source unit 33, rather than irradiating X-rays. That is, the operator performs an operation to turn on the light source unit 33 after the rough position adjustment of the subject 101 is completed. Therefore, by configuring as described above, the operator can naturally perform an operation (pressing the input receiving unit 43) that triggers switching between the first mode and the second mode during a series of operations of performing position adjustment of the X-ray irradiation unit 10 and adjustment of the X-ray irradiation field by the collimator unit 12 after performing the rough position adjustment of the subject 101. As a result, since it is possible to make the operator perform the operation of switching between the first mode and the second mode smoothly during the operator's position adjustment work, it is possible to suppress a decrease in workability of position adjustment by the operator.

Furthermore, in the first embodiment, as described above, the optical imaging control unit 32 (control unit) is configured to perform control to superimpose the detection unit region display 81, the X-ray irradiation field region display 84, and the AEC region display 87 (X-ray light receiving field region display) as the region display 80 on the optical image 70 to display on the display operation unit 41 (display unit) when displaying the optical image 70 in the second mode. Thus, the operator can confirm the arrangement of the X-ray irradiation field relative to a region where X-rays are detected, by visually recognizing the optical image 70 on which the detection unit region display 81, the X-ray irradiation field region display 84, and the AEC region display 87 are superimposed. Therefore, in addition to the arrangement of the subject 101 in the region where X-rays are detected, the arrangement of the X-ray irradiation field relative to the region where X-rays are detected can also be confirmed based on the optical image 70 on which the detection unit region display 81, the X-ray irradiation field region display 84, and the AEC region display 87 are superimposed. Further, when adjusting the irradiation field of X-rays irradiated from the X-ray tube 11 of the X-ray irradiation unit 10 by the collimator unit 12, the operator can easily adjust the irradiation field of X-rays by the collimator unit 12 by visually recognizing the X-ray irradiation field region display 84 displayed superimposed on the optical image 70.

Moreover, in the first embodiment, as described above, the optical imaging control unit 32 (control unit) is configured to perform control to make the display aspect of the region display 80 in the first mode and the display aspect of the region display 80 in the second mode mutually different, and to superimpose it on the optical image 70 and display it on the display operation unit 41 (display unit). As a result, since the display aspects of the region display 80 differ from each other depending on the first mode and the second mode, the operator can easily visually recognize whether the optical image 70 in the first mode is displayed or the optical image 70 in the second mode is displayed on the display operation unit 41. Further, since the region display 80 in the first mode is displayed based on a predetermined fixed position in the real space preset in the X-ray irradiation direction in a state where the X-ray irradiation unit 10 and the X-ray detection unit 20 face each other, for example, by displaying it with a lighter color, it is possible to make the operator visually recognize that accuracy of the position and shape of the region display 80 is low. Furthermore, for example, by displaying the region display 80 in the second mode with a darker color, it is possible to make the operator visually recognize that accuracy of the position and shape of the region display 80 is high. As a result of these, convenience (usability) for the operator can be improved.

Further, in the first embodiment, as described above, the optical imaging control unit 32 (control unit) is configured to acquire the body thickness of the subject 101 based on any one of the optical image 70 captured by the optical imaging unit 31, an operator's selection operation of body thickness information of the subject 101, and the body type information 102 of the subject 101, and to perform control to display the optical image 70 superimposed with the region display 80 deformed according to the acquired body thickness of the subject 101 based on the position on the body surface 101a of the subject 101 in the real space on the display operation unit 41 (display unit) in the second mode. As a result, when the body thickness of the subject 101 is acquired based on the optical image 70 captured by the optical imaging unit 31, the body thickness of the subject 101 can be acquired without the operator performing an operation input by capturing the optical image 70 by the optical imaging unit 31. Moreover, also in a case where the body thickness of the subject 101 is acquired based on the body type information 102 of the subject 101, the body thickness of the subject 101 can be acquired without the operator performing a selection operation. As a result, an increase in a burden on the operator can be suppressed. Further, when the body thickness of the subject 101 is acquired by a selection operation of the body thickness information of the subject 101 by the operator, the body thickness of the subject 101 can be acquired without calculating the body thickness of the subject 101. As a result, compared with a case where the optical imaging control unit 32 acquires the body thickness of the subject 101, processing load of the optical imaging control unit 32 can be reduced.

Moreover, in the first embodiment, as described above, the optical imaging control unit 32 (control unit) is configured to perform control to display the optical image 70 superimposed with the region display 80 based on the fixed position on the display operation unit 41 (display unit), based on the position information 103 of the X-ray detection unit 20 or the standard body thickness information 104 preset for each imaging region of the subject 101, when superimposing the region display 80 on the optical image 70 to display it in the first mode. Thus, when superimposing the region display 80 based on the fixed position on the optical image 70 based on the position information 103 of the X-ray detection unit 20, the region display 80 can always be displayed at the position of the X-ray detection unit 20 regardless of the imaging region of the subject 101. Further, since the position information 103 of the X-ray detection unit 20 can be acquired by the operation detection unit 50b, the region display 80 can be easily superimposed on the position of the X-ray detection unit 20. Furthermore, when superimposing the region display 80 based on the fixed position on the optical image 70 based on the standard body thickness information 104, the region display 80 can be superimposed at a position corresponding to an average body thickness according to an imaging region using the standard body thickness information 104 which is information of body thickness based on a statistical value (average value) for each imaging region of the subject 101. In addition, when imaging the subject 101 with the X-ray imaging apparatus 100, the operator performs an operation of selecting the imaging region of the subject 101. Therefore, based on the operator's operation of selecting the imaging region, it becomes possible to acquire the standard body thickness information 104 according to the imaging region, so that the region display 80 can be easily superimposed at a position corresponding to the average body thickness according to the imaging region.

Further, in the first embodiment, as described above, the holding unit 40 holding the X-ray irradiation unit 10 is further provided, and the optical imaging unit 31 is provided on the holding unit 40 together with the X-ray irradiation unit 10. Consequently, since the X-ray irradiation unit 10 and the optical imaging unit 31 are both provided on the holding unit 40, an irradiation direction of X-rays irradiated from the X-ray irradiation unit 10 and an imaging direction of the optical imaging unit 31 can be aligned. Therefore, position adjustment of the subject 101 and position adjustment of the X-ray irradiation unit 10 can be easily performed while referring to the optical image 70 captured by the optical imaging unit 31.

### [Second Embodiment]

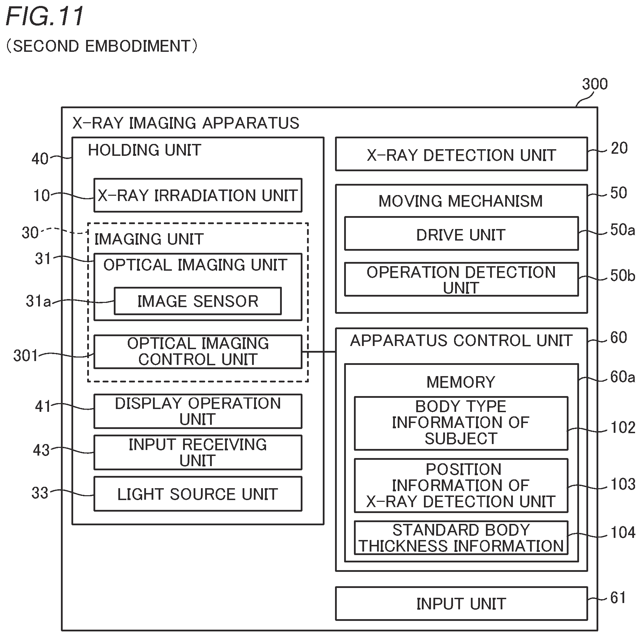

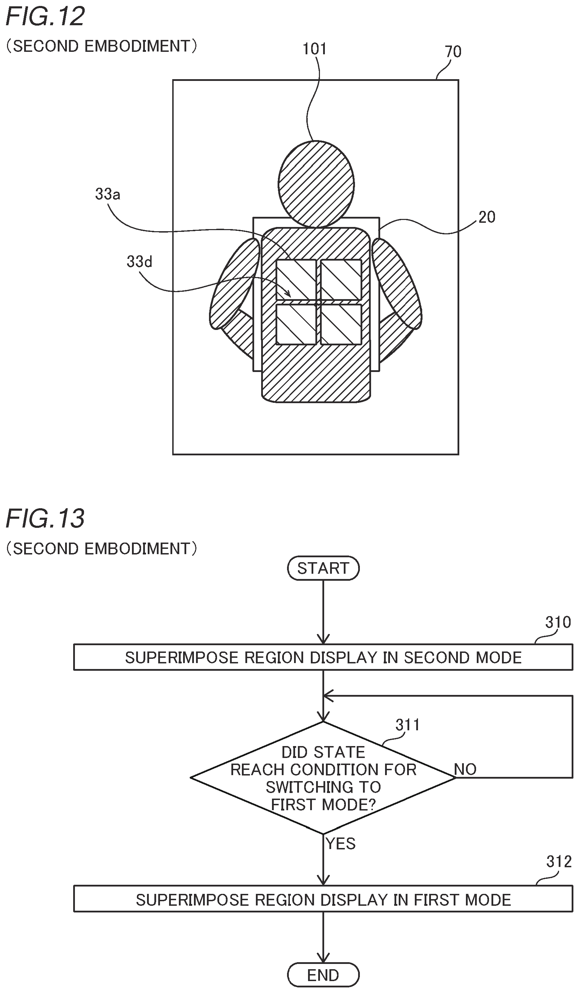

Next, an X-ray imaging apparatus 300 according to a second embodiment will be described with reference to Figs. 11 and 12.

The X-ray imaging apparatus 300 according to the second embodiment is different from the X-ray imaging apparatus 100 according to the first embodiment described above in that it includes an optical imaging control unit 301 instead of the optical imaging control unit 32.

The optical imaging control unit 301 is configured to switch a mode for displaying the optical image 70 with the region display 80 superimposed thereon from a second mode to a first mode, based on an input from an operator received by the input receiving unit 43 or the optical image 70.

Specifically, the optical imaging control unit 301 switches from the second mode to the first mode when completion of imaging or a variation in a position of the body surface of the subject 101 is detected based on an input from the operator received by the input receiving unit 43. Also, the optical imaging control unit 301 is configured to switch from the second mode to the first mode based on a variation in the position of the body surface of the subject 101 acquired based on the optical image 70, or a crosshair 33d (see Fig. 12) shown in the optical image 70.

Here, after imaging of a predetermined imaging region of the subject 101 is completed, imaging of another imaging region may be subsequently performed. When imaging another imaging region continuously, position adjustment of the subject 101 is performed again. Further, when imaging a different subject after imaging of the subject 101 is completed, position adjustment of the different subject is also performed. That is, after imaging of the subject 101 is completed, position adjustment of the subject is performed again. When position adjustment of the subject is performed again and the mode for superimposing the region display 80 on the optical image 70 for display is the second mode, a position and a shape of the region display 80 change frequently during position adjustment of the subject 101. In this case, work efficiency of position adjustment decreases due to the position and shape of the region display 80 changing frequently during display.

Therefore, in the second embodiment, the optical imaging control unit 301 switches from the second mode to the first mode when imaging of the subject 101 is completed. Note that the optical imaging control unit 301 determines whether or not imaging has been completed by receiving an input for X-ray irradiation by the operator via the input receiving unit 43, or by the optical imaging control unit 301 acquiring X-ray irradiation. Further, the optical imaging control unit 301 may determine that imaging is completed based on an operation input indicating completion of imaging being performed by the operator.

Also, for example, if body movement occurs in the subject 101 after position adjustment of the subject 101 is completed, position adjustment of the subject 101 may be performed again. At this time as well, if the mode for displaying the optical image 70 with the region display 80 superimposed thereon is the second mode, work efficiency of position adjustment decreases due to the position and shape of the region display 80 changing frequently during display.

Therefore, in the second embodiment, the optical imaging control unit 301 is configured to switch from the second mode to the first mode based on a variation in the position of the body surface 101a of the subject 101. Specifically, the optical imaging control unit 301 switches from the second mode to the first mode when a range of variation of an X-ray tube-subject distance D1 (see Fig. 9), which is a distance between the X-ray irradiation unit 10 and the body surface 101a of the subject 101, falls outside a predetermined range. Note that the optical imaging control unit 301 may be configured to switch from the second mode to the first mode based on an operation input performed by the operator indicating that a variation in the position of the body surface 101a of the subject 101 has become large.

Moreover, similar to the optical imaging control unit 32 according to the first embodiment described above, the optical imaging control unit 301 calculates an X-ray tube-subject distance D1 between the X-ray tube 11 (focal point thereof) and the subject 101 based on coordinates of a center as a predetermined position 33c (see Fig. 8) in an irradiation field region 33a (see Fig. 4). At this time, as shown in Fig. 12, the optical imaging control unit 301 uses coordinates of a center of a crosshair 33d in the irradiation field region 33a as the predetermined position 33c for calculating the X-ray tube-subject distance D1. When a contrast of the crosshair 33d in the irradiation field region 33a is low, it becomes difficult to accurately acquire a center position of the irradiation field region 33a. Therefore, it becomes difficult for the optical imaging control unit 301 to accurately acquire the X-ray tube-subject distance D1. If it is difficult to accurately acquire the X-ray tube-subject distance D1, it becomes difficult to accurately superimpose the region display 80 on the body surface 101a of the subject 101 in the optical image 70. Therefore, in the second embodiment, the optical imaging control unit 301 is configured to switch from the second mode to the first mode when the contrast of the crosshair 33d in the irradiation field region 33a decreases.

Next, with reference to Fig. 13, processing in which the optical imaging control unit 301 (see Fig. 11) switches the mode for displaying the region display 80 (see Fig. 5) superimposed on the optical image 70 (see Fig. 5) from the second mode to the first mode will be described.

In step 310, the optical imaging control unit 301 superimposes the region display 80 on the optical image 70 and displays it by the second mode. Note that since processing in step 310 is similar to the processing from step 201 to step 208 shown in Fig. 10 performed by the optical imaging control unit 32 according to the first embodiment described above, detailed description thereof is omitted.

Next, in step 311, the optical imaging control unit 301 determines whether or not a state has reached a condition for switching to the first mode. Specifically, the optical imaging control unit 301 determines whether or not to switch to the first mode based on an input from the operator received by the input receiving unit 43, or the optical image 70. If switching to the first mode, processing proceeds to step 312. If not switching to the first mode, the optical imaging control unit 301 repeats the processing of step 311.

In step 312, the optical imaging control unit 301 switches the mode for displaying the region display 80 superimposed on the optical image 70 from the second mode to the first mode. Thereafter, the processing ends.

Note that other configurations of the second embodiment are similar to the configurations of the first embodiment described above.

### (Effects of Second Embodiment)

In the second embodiment, as described above, the optical imaging control unit 301 is configured to switch the mode for displaying the region display 80 superimposed on the optical image 70 from the second mode to the first mode, based on an input from the operator received by the input receiving unit 43 or the optical image 70. Consequently, when a situation requiring switching from the second mode to the first mode arises, such as when another imaging region is to be imaged consecutively after completion of imaging, when position adjustment of the subject 101 is to be performed again, or when the contrast of the crosshair 33d in the irradiation field region 33a shown in the optical image 70 decreases, the mode in which the region display 80 is superimposed can be switched from the second mode to the first mode without the operator performing a mode switching operation. As a result, compared to a configuration in which switching from the second mode to the first mode is not performed even when a situation requires switching from the second mode to the first mode, it is possible to prevent a decrease in work efficiency of position adjustment caused by frequent changes in the position and shape of the region display 80 during display.

Note that other effects of the second embodiment are similar to the effects of the first embodiment described above.

### [Third Embodiment]

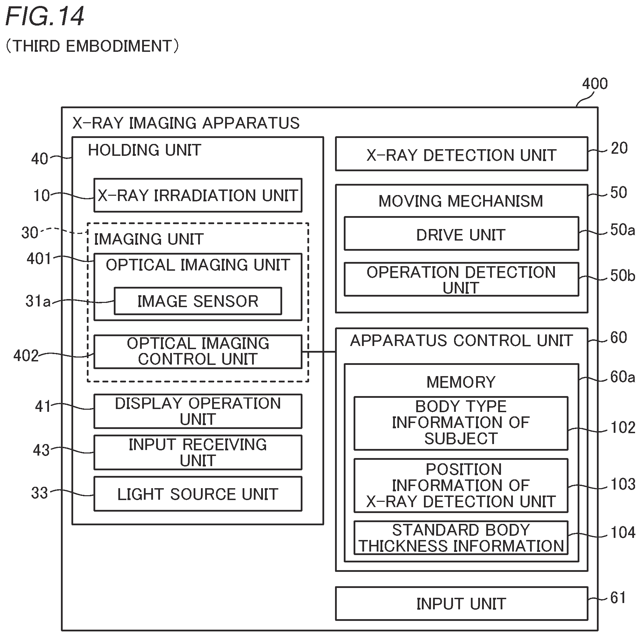

Next, an X-ray imaging apparatus 400 according to a third embodiment will be described with reference to Fig. 14.

The X-ray imaging apparatus 400 according to the third embodiment differs from the X-ray imaging apparatus 100 according to the first embodiment described above in that an optical imaging unit 401 and an optical imaging control unit 402 are provided instead of the optical imaging unit 31 and the optical imaging control unit 32.

The optical imaging unit 401 is different from the optical imaging unit 31 according to the first embodiment described above in that it acquires a position of a body surface 101a of a subject 101 in a real space. Note that the optical imaging unit 401 is an example of a "body surface position acquisition unit" in the claims. In the third embodiment, the optical imaging unit 401 has both functions of an optical imaging unit for capturing an optical image 70 of the subject 101, and a body surface position acquisition unit for acquiring a position of the body surface 101a of the subject 101 in a real space. Note that the X-ray imaging apparatus 400 according to the third embodiment may include a 3D sensor capable of measuring a position in a depth direction as the body surface position acquisition unit.

Further, the optical imaging control unit 402 according to the third embodiment is configured to perform control to switch from a first mode of indicating a region where an X-ray detection unit 20 is arranged in the optical image 70, based on an input from an operator received by an input receiving unit 43 or a position of the body surface 101a of the subject 101 acquired by the optical imaging unit 401, with reference to a predetermined fixed position in a real space, displaying the optical image 70 superimposed with a region display 80 including at least any one of a detection unit region display 81 indicating a region where an X-ray irradiation unit 10 is arranged in the optical image 70, an X-ray irradiation field region display 84 indicating a region to which X-rays are irradiated by the X-ray irradiation unit 10 in the optical image 70, and an AEC region display 87 indicating a region receiving X-rays irradiated by the X-ray irradiation unit 10 in the optical image 70, or displaying the optical image 70 with the region display 80 hidden, without superimposing the region display 80 on the optical image 70, to a second mode of displaying the optical image 70 superimposed with the region display 80 based on a position on the body surface 101a of the subject 101 in the real space. That is, the optical imaging control unit 402 according to the third embodiment is configured to be capable of executing both manually switching from the first mode to the second mode by an operation input by the operator, and automatically switching from the first mode to the second mode based on a position of the body surface 101a of the subject 101 acquired by the optical imaging unit 401.

Note that a configuration in which the optical imaging control unit 402 switches from the first mode to the second mode based on an input from the operator received by the input receiving unit 43 is similar to the configuration in which the optical imaging control unit 32 according to the first embodiment described above switches from the first mode to the second mode based on an input from the operator received by the input receiving unit 43, and therefore a detailed description is omitted.

Therefore, in the third embodiment, a configuration in which the optical imaging control unit 402 switches from the first mode to the second mode based on the position of the body surface 101a of the subject 101 acquired by the optical imaging unit 401 will be described.

Here, when position adjustment of the subject 101 is completed, the position of the body surface 101a of the subject 101 substantially stops moving at a predetermined position. Therefore, in the third embodiment, the optical imaging control unit 402 is configured to switch from the first mode to the second mode based on a position of the body surface 101a of the subject 101 appearing in the optical image 70.

In the third embodiment, the optical imaging control unit 402 acquires an X-ray tube-subject distance D1 based on the position of the body surface 101a of the subject 101 appearing in the optical image 70. Note that since a configuration in which the optical imaging control unit 402 acquires the X-ray tube-subject distance D1 is similar to the configuration in which the optical imaging control unit 32 according to the first embodiment described above acquires the X-ray tube-subject distance D1, a detailed description thereof is omitted. The optical imaging control unit 402 is configured to switch from the first mode to the second mode based on an amount of variation in the acquired X-ray tube-subject distance D1. Specifically, the optical imaging control unit 402 switches from the first mode to the second mode when the amount of variation in the X-ray tube-subject distance D1 falls within a predetermined range.

Note that the optical imaging control unit 402 may be configured to switch from the first mode to the second mode based on the subject 101 appearing in the optical image 70. That is, the optical imaging control unit 402 may determine whether or not position adjustment of the subject 101 is completed by analyzing the image of the subject 101 appearing in the optical image 70, and may be configured to switch from the first mode to the second mode when the position adjustment of the subject 101 is completed.

Also, if a position of the X-ray irradiation unit 10 varies after the position adjustment of the subject 101 is completed, the X-ray tube-subject distance D1 also varies. Therefore, the optical imaging control unit 402 is configured to switch from the second mode to the first mode when the amount of variation in the X-ray tube-subject distance D1 falls outside the predetermined range due to movement of the X-ray irradiation unit 10, after the amount of variation in the X-ray tube-subject distance D1 falls within a predetermined range. Note that the X-ray irradiation unit 10 is provided with a sensor (such as a potentiometer) capable of acquiring a movement amount of the X-ray irradiation unit 10, so that the optical imaging control unit 402 can acquire the movement amount of the X-ray irradiation unit 10.

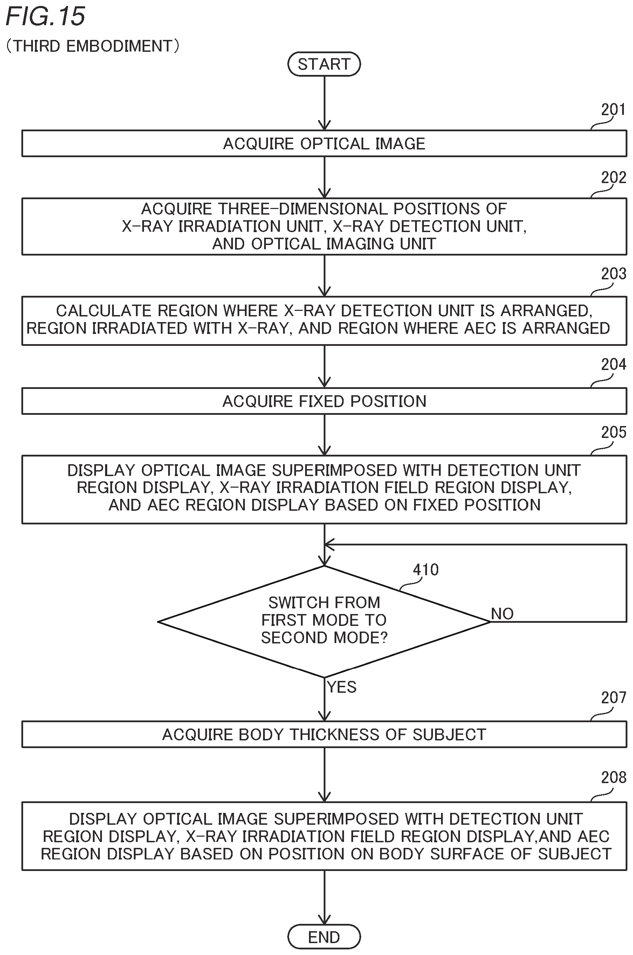

Next, referring to Fig. 15, control processing of an image display method by the X-ray imaging apparatus 400 (see Fig. 14) of the third embodiment will be described. The control processing of the image display method by steps 201 to 205, step 410, step 207, and step 208 is executed by the optical imaging control unit 402 (see Fig. 14). Note that processing similar to the processing performed by the optical imaging control unit 32 according to the first embodiment described above is denoted by the same reference numerals, and a detailed description thereof is omitted.

In steps 201 to 205, the optical imaging control unit 402 displays the optical image 70 superimposed with the region display 80 (detection unit region display 81, X-ray irradiation field region display 84, and AEC region display 87) based on a predetermined fixed position in the real space on the display operation unit 41. That is, the optical imaging control unit 402 sets the mode for superimposing the region display 80 on the optical image 70 and displaying it to the first mode.

Next, in step 410, the optical imaging control unit 402 determines whether to switch from the first mode to the second mode. Specifically, the optical imaging control unit 402 determines whether to switch from the first mode to the second mode based on a position of the body surface 101a of the subject 101 acquired by the optical imaging unit 401. If switching from the first mode to the second mode, the processing proceeds to step 207. If not switching from the first mode to the second mode, the optical imaging control unit 402 repeats the processing of step 410.

Next, in steps 207 and 208, the optical imaging control unit 402 displays the optical image 70 superimposed with the region display 80 (detection unit region display 81, X-ray irradiation field region display 84, and AEC region display 87) based on a position on the body surface 101a of the subject 101 in the real space on the display operation unit 41. That is, the optical imaging control unit 402 switches the mode for superimposing the region display 80 on the optical image 70 and displaying it from the first mode to the second mode. Thereafter, the processing ends.

The processing in which the optical imaging control unit 402 switches from the first mode to the second mode based on an input from the operator received by the input receiving unit 43 is similar to the processing performed by the optical imaging control unit 32 according to the first embodiment described above based on the flowchart shown in Fig. 10, and thus detailed description is omitted.

Note that other configurations of the third embodiment are similar to the configurations of the first embodiment described above.

### (Effects of Third Embodiment)

In the third embodiment, as described above, the X-ray imaging apparatus 400 includes the X-ray irradiation unit 10 including the X-ray tube 11, the X-ray detection unit 20 configured to detect X-rays irradiated from the X-ray irradiation unit 10 and passed through the subject 101, the optical imaging unit 401 configured to capture an optical image 70 of the subject 101 and acquire a position of a body surface 101a of the subject 101 in a real space, the display operation unit 41 configured to display the optical image 70 captured by the optical imaging unit 401, the optical imaging control unit 402 configured to perform control to display the optical image 70 on the display operation unit 41, and the input receiving unit 43 configured to receive an input from the operator. The optical imaging control unit 402 is configured to perform control to switch from the first mode of indicating a region where an X-ray detection unit 20 is arranged in the optical image 70, based on an input from an operator received by the input receiving unit 43 or a position of the body surface 101a of the subject 101 acquired by the optical imaging unit 401, with reference to a predetermined fixed position in a real space, displaying the optical image 70 superimposed with the region display 80 including at least any one of a detection unit region display 81 indicating a region where an X-ray irradiation unit 10 is arranged in the optical image 70, an X-ray irradiation field region display 84 indicating a region to which X-rays are irradiated by the X-ray irradiation unit 10 in the optical image 70, and an AEC region display 87 indicating a region receiving X-rays irradiated by the X-ray irradiation unit 10 in the optical image 70, or displaying the optical image 70 with the region display 80 hidden, without superimposing the region display 80 on the optical image 70, to the second mode of displaying the optical image 70 superimposed with the region display 80 based on a position on the body surface 101a of the subject 101 in the real space, when position adjustment is performed.

Thus, when the optical imaging control unit 402 switches from the first mode to the second mode based on an input from the operator received by the input receiving unit 43, it is possible to provide the X-ray imaging apparatus 400 capable of easily performing position adjustment while suppressing a decrease in work efficiency, similar to the X-ray imaging apparatus 100 according to the first embodiment described above. Also, when the optical imaging control unit 402 switches from the first mode to the second mode based on a position of the body surface 101a of the subject 101 acquired by the optical imaging unit 401, it switches from the first mode to the second mode based on the position of the body surface 101a of the subject 101 acquired by the optical imaging unit 401, so that the mode can be automatically switched from the first mode to the second mode without the operator performing an operation input. As a result, it is possible to provide the X-ray imaging apparatus 400 capable of easily performing position adjustment while simultaneously achieving a reduction in work burden of the operator and a suppression of a decrease in work efficiency.

Note that other effects of the third embodiment are similar to the effects of the first embodiment described above.

### [Modifications]

It should be understood that the embodiments disclosed this time are illustrative in all respects and are not restrictive. The scope of the present invention is shown by the claims rather than the description of the above embodiments, and further includes all modifications (modified examples) within the meaning and scope equivalent to the claims.

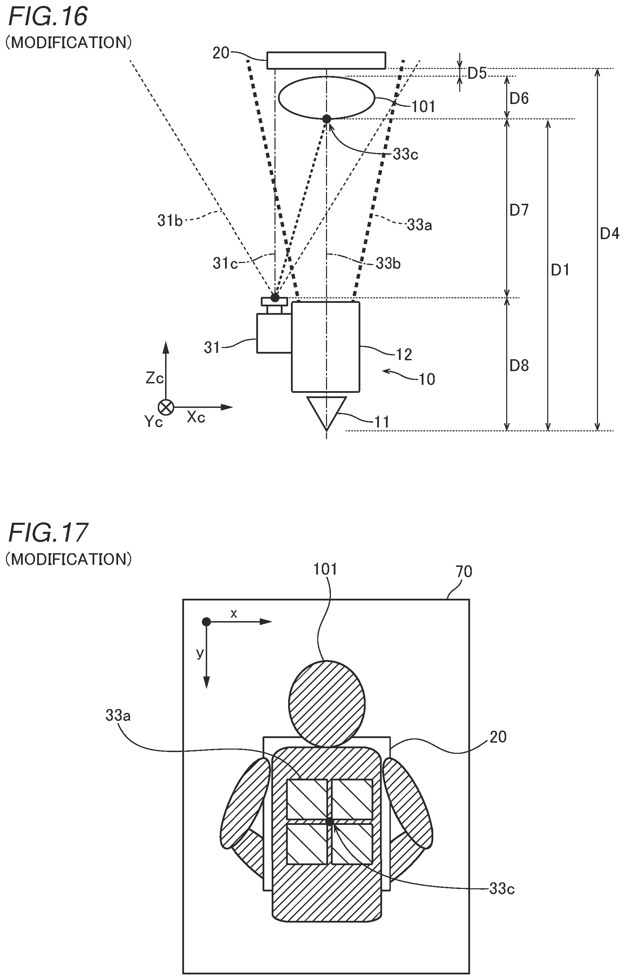

For example, in the first to third embodiments described above, an example was shown in which the optical imaging control unit 32 (control unit) calculates a body thickness of a subject 101 based on a coordinate difference D2 between coordinates of a center as a predetermined position 20a of the X-ray detection unit 20 in an optical image 70 and coordinates of a center as a predetermined position 33c in an irradiation field region 33a appearing on a body surface of the subject 101 in the optical image 70, but the present invention is not limited to this. In the present invention, for example, a control unit (optical imaging control unit) may be configured to calculate a body thickness of a subject 101 based on coordinates of a center as a predetermined position 33c in an irradiation field region 33a appearing on a body surface of the subject 101 in an optical image 70, as shown in modifications in Figs. 16 and 17.

Specifically, a control unit (optical imaging control unit) calculates an imaging unit-body surface distance D7 between an optical imaging unit 31 and a body surface of a subject 101 in an optical axis direction of a light source unit 33 based on coordinates of a center as a predetermined position 33c in an irradiation field region 33a appearing on the body surface of the subject 101 in an optical image 70, coordinates in an imaging unit coordinate system centered on the optical imaging unit 31, and a relational expression for converting coordinates in the imaging unit coordinate system into coordinates in an image coordinate system indicating coordinates in the optical image 70 (a relational expression using a matrix of internal parameters of the optical imaging unit 31). Then, the control unit (optical imaging control unit) calculates an X-ray tube-subject distance D1 based on the calculated imaging unit-body surface distance D7 and an X-ray tube-imaging unit distance D8 between an X-ray tube 11 and the optical imaging unit 31 in the optical axis direction of the light source unit 33. Note that the X-ray tube-imaging unit distance D8 can be calculated by an arrangement of the optical imaging unit 31 in an X-ray irradiation unit 10.

Further, assuming an irradiation axis direction of the X-ray irradiation unit 10 to be a Z direction, and mutually orthogonal directions in a plane orthogonal to the Z direction to be an X direction and a Y direction, respectively, and coordinates of a center as a predetermined position 33c in the irradiation field region 33a appearing on the body surface of the subject 101 in the optical image 70 to be coordinates (Xc, Yc, Zc) in an imaging unit coordinate system centered on the optical imaging unit 31 (origin) in a real space, and to be coordinates (xh, yh) in an image coordinate system (x, y) indicating coordinates in the optical image 70, as (Xch, Ych, Zch) and (xh, yh), respectively, the following formula (1) holds.
[Formula 1] $\left[\begin{matrix}{x}_{h} \\ {y}_{h} \\ 1\end{matrix}\right] = K \left[\begin{matrix}{Xc}_{h} / {Zc}_{h} \\ {Yc}_{h} / {Yc}_{h} \\ 1\end{matrix}\right] = \left[\begin{matrix}\frac{w}{Sx} \times f & 0 & w / 2 \\ 0 & \frac{h}{Sy} \times f & h / 2 \\ 0 & 0 & 1\end{matrix}\right] \left[\begin{matrix}{Xc}_{h} / {Zc}_{h} \\ {Yc}_{h} / {Yc}_{h} \\ 1\end{matrix}\right]$

Here, K is a matrix for converting coordinates in the imaging unit coordinate system to coordinates in the image coordinate system, which is called a matrix of internal parameters of the optical imaging unit 31. Namely, f is a focal length of a lens of the optical imaging unit 31, (Sx, Sy) is a size of an image sensor 31a of the optical imaging unit 31, and (w, h) is the number of pixels of the image sensor 31a of the optical imaging unit 31.

From the above formula (1), the following formulas (2) and (3) are derived.
[Formula 2] ${x}_{h} = \frac{w}{Sx} \times f \times \frac{{Xc}_{h}}{{Zc}_{h}} + \frac{w}{2}$
[Formula 3] ${y}_{h} = \frac{h}{Sy} \times f \times \frac{{Yc}_{h}}{{Zc}_{h}} + \frac{h}{2}$

From the above formula (2) and formula (3), the following formula (4) and formula (5) are derived, respectively.
[Formula 4] ${Zc}_{h} = \frac{\frac{w}{Sx} \times f \times {Xc}_{h}}{{x}_{h} - \frac{w}{2}}$
[Formula 5] ${Zc}_{h} = \frac{\frac{h}{Sy} \times f \times {Yc}_{h}}{{y}_{h} - \frac{h}{2}}$

Thereby, from the above formula (4) or formula (5), Zch can be calculated. Zch is a Z direction component of coordinates in an imaging unit coordinate system centered on the optical imaging unit 31 (origin) corresponding to coordinates of a center as a predetermined position 33c in an irradiation field region 33a appearing on a body surface of a subject 101 in an optical image 70. Zch is equal to an imaging unit-body surface distance D7.

Then, a control unit (optical imaging control unit) adds the imaging unit-body surface distance D7 and an X-ray tube-imaging unit distance D8 between an X-ray tube 11 and the optical imaging unit 31 in an optical axis direction of a light source unit 33 to calculate an X-ray tube-subject distance D1. Then, the control unit (optical imaging control unit) acquires a body thickness of the subject 101 by calculating a distance D6 from the X-ray tube-subject distance D1, an X-ray tube-detector distance D4, and a detector-body back surface distance D5.

Note that in the first to third embodiments and the modification example described above, an example was shown in which an optical imaging control unit 32 (control unit) calculates a body thickness of a subject 101 from an optical image 70 in a state where an optical axis 31c of an optical imaging unit 31 and an optical axis 33b of a light source unit 33 are parallel to each other, but the present invention is not limited to this. In the present invention, for example, if the optical axis 31c of the optical imaging unit 31 and the optical axis 33b of the light source unit 33 are not parallel to each other, the control unit (optical imaging control unit) may be configured to calculate a body pressure of the subject 101 based on the optical image 70 taking an angle between the optical axis 31c of the optical imaging unit 31 and the optical axis 33b of the light source unit 33 into consideration (an inclination of the optical axis 31c of the optical imaging unit 31 with respect to the optical axis 33b of the light source unit 33).

Further, in the first to third embodiments described above, an example was shown in which an optical imaging control unit 32 (control unit) including a microcomputer is configured to superimpose a region display 80 (a detection unit region display 81, an X-ray irradiation field region display 84, and an AEC region display 87 (X-ray light receiving field region)) on an optical image 70 to display on a display operation unit 41 (display unit), but the present invention is not limited to this. In the present invention, an apparatus control unit that controls X-ray imaging may be configured to superimpose a region display (a detection unit region display, an X-ray irradiation field region display, and an AEC region display) on an optical image for display. Further, an optical image on which a region display is superimposed by a control unit (optical imaging control unit) may be output to the outside, and simultaneously the region display may be superimposed on the optical image to be displayed on a display unit arranged at a position separated from the control unit. For example, a display unit may be provided on an imaging stand, and the optical image superimposed with the region display may be output and displayed on the display unit provided on the imaging stand. Furthermore, for example, the optical image superimposed with the region display may be displayed on a display unit provided on an apparatus control unit arranged outside an imaging room. Also, a control unit may be configured by a personal computer, a processor, or a circuitry. Furthermore, each control processing by a control unit may be executed by a combination of different hardware.

Further, in the first to third embodiments described above, an example was shown in which an optical imaging control unit 32 is configured to superimpose a detection unit region display 81, an X-ray irradiation field region display 84, and the AEC region display 87 (X-ray light receiving field region) as a region display 80 on an optical image 70 for display, but the present invention is not limited to this. In the present invention, an optical imaging control unit (control unit) may be configured to superimpose at least any one of a detection unit region display, an X-ray irradiation field region display, and an AEC region display on an optical image for display as a region display.

Further, in the first to third embodiments described above, an example was shown in which an optical imaging control unit 32 superimposes a region display 80 deformed according to a body thickness of a subject 101 based on a position on a body surface 101a of the subject 101 in a real space when displaying the region display 80 superimposed on an optical image 70 in a second mode, but the present invention is not limited to this. In the present invention, an optical imaging control unit (control unit) may not deform a shape of a region display according to a body thickness of a subject when superimposing the region display in a second mode. However, if the shape of the region display is not deformed according to the body thickness of the subject when superimposing the region display in the second mode, it becomes difficult for an operator to visually recognize an accurate size of an X-ray detection unit in an optical image. Therefore, it is preferable that an optical imaging control unit (control unit) is configured to superimpose a region display deformed according to a body thickness of a subject onto a body surface of the subject when displaying the region display superimposed on an optical image in a second mode.

Further, in the first to third embodiments described above, an example was shown in which an optical imaging control unit 32 displays a mode switching button 44 and a body thickness level selection button 45 on a display operation unit 41, but the present invention is not limited to this. In the present invention, an optical imaging control unit (control unit) may not display a mode switching button and a body thickness level selection button on a display operation unit (display unit). If an optical imaging control unit (control unit) does not display a mode switching button on a display operation unit (display unit), the optical imaging control unit may be configured to switch between a first mode and a second mode based on an operation input to an input receiving unit. Furthermore, if an optical imaging control unit (control unit) does not display a body thickness level selection button on a display operation unit (display unit), the optical imaging control unit may be configured to acquire a body thickness of a subject based on an optical image, or to acquire a body thickness of a subject based on body type information of the subject.

Further, in the first to third embodiments described above, an example was shown in which an optical imaging control unit 32 switches between a first mode and a second mode based on an input by an input receiving unit 43, but the present invention is not limited to this. In the present invention, an optical imaging control unit (control unit) may be configured to switch between a first mode and a second mode based on, for example, a gesture or a vocalization of an operator. When switching between a first mode and a second mode by an operator's gesture, an optical imaging control unit (control unit) may be configured to determine whether or not an operator performs a predetermined gesture in an image (video image) captured by an optical imaging unit 31. Further, when switching between a first mode and a second mode by an operator's vocalization, an X-ray imaging apparatus may include a sound collector (microphone) that detects the operator's vocalization, and an optical imaging control unit (control unit) may be configured to determine whether or not the operator utters a predetermined keyword.

Further, in the first to third embodiments described above, although an example of a configuration was shown in which an optical imaging control unit 32 calculates a body thickness of a subject 101 based on a height and a weight of the subject 101 when calculating the body thickness of the subject 101 based on body type information 102 of the subject 101, the present invention is not limited to this. In the present invention, an optical imaging control unit (control unit) may be configured to calculate a body thickness of a subject based on an age and/or a gender of the subject together with a height and a weight of the subject when calculating the body thickness of the subject based on body type information of the subject.

Further, in the first to third embodiments described above, an example was shown in which an optical imaging control unit 32 displays an SID display region 90, an SOD display region 91, and an irradiation unit angle display region 92 on a display operation unit 41, but the present invention is not limited to this. In the present invention, an optical imaging control unit (control unit) may not display an SID display region, an SOD display region, and an irradiation unit angle display region on a display operation unit (display unit).

Further, in the first to third embodiments described above, an example was shown in which an imaging unit 30 including an optical imaging unit 31 and an optical imaging control unit 32 (control unit) is arranged in a holding unit 40 holding an X-ray irradiation unit 10, but the present invention is not limited to this. In the present invention, an optical imaging unit and a control unit may be arranged at positions separated from each other. Also, an optical imaging unit may be arranged at a position different from a holding unit, such as a ceiling or a wall surface of an imaging room. Furthermore, an imaging unit may be configured as an add-on unit to an X-ray imaging apparatus.

Further, in the first to third embodiments described above, an example was shown in which a position and an angle of an X-ray irradiation unit 10 and a position of an X-ray detection unit 20 are acquired based on a detection signal from an operation detection unit 50b in a moving mechanism 50 that moves the X-ray irradiation unit 10 and the X-ray detection unit 20, but the present invention is not limited to this. In the present invention, a relative positional relationship between an X-ray detection unit and an X-ray irradiation unit may be acquired by providing a detection unit such as a magnetic sensor on at least one of the X-ray detection unit and the X-ray irradiation unit. Furthermore, a region where an X-ray detection unit is arranged may be detected by performing image processing on an optical image. For example, a marker member indicating a position of an X-ray detection unit may be arranged together with the X-ray detection unit, and a region where the X-ray detection unit is arranged may be detected by detecting the marker member from a captured optical image.

Further, in the first to third embodiments described above, an example was shown in which a detection unit region display 81 has a cross-shaped center display 82 and four region displays 83 indicating four corners, and an X-ray irradiation field region display 84 has a region display 85 which is a rectangular frame line and a pair of dotted line displays 86, but the present invention is not limited to this. In the present invention, a detection unit region display and an X-ray irradiation field region display may be displayed in an aspect different from those in the first to third embodiments. For example, either a detection unit region display or an X-ray irradiation field region display may not include a display indicating a center of a region. Further, a range of a region may be indicated by a rectangular frame line in a detection unit region display. In an X-ray irradiation field region display, only a rectangular frame line may be shown without showing a pair of dotted line displays, or a range of a rectangular region may be shown only by displaying four corners. At least one of a detection unit region display and an X-ray irradiation field region display may be displayed transparently, or may be displayed in a blinking manner.

Further, in the first to third embodiments described above, the process in which an optical imaging control unit 32 (301, 402) switches a mode for displaying a region display 80 superimposed on an optical image 70 from a first mode to a second mode, and the process in which the optical imaging control unit 301 switches the mode for displaying the region display 80 superimposed on the optical image 70 from the second mode to the first mode were described using a flow-driven flowchart in which processing is performed sequentially along a processing flow, but the present invention is not limited to this. For example, in the present invention, each process described above by a control unit may be performed by an event-driven process that executes processing on an event basis. In this case, it may be performed completely in an event-driven manner, or it may be performed by combining event-driven and flow-driven processing.

Further, an example was shown in which each of the first to third embodiments described above is an individual embodiment, but the first and second embodiments may be combined together to form a configuration, or the second and third embodiments may be combined together to form a configuration.

### [Aspects]

It will be understood by those skilled in the art that the exemplary embodiments described above are specific examples of the following aspects.

### (Item 1)

An X-ray imaging apparatus, comprising:
an X-ray irradiation unit including an X-ray tube;
an X-ray detection unit that detects X-rays irradiated from the X-ray irradiation unit and passed through a subject;
an optical imaging unit that captures an optical image of the subject;
a display unit that displays the optical image captured by the optical imaging unit;
a control unit that performs control to display the optical image on the display unit; and an input receiving unit that receives an input from an operator,
wherein the control unit is configured to switch from a first mode of, when performing position adjustment, based on the input from the operator received by the input receiving unit, with reference to a predetermined fixed position in a real space, displaying the optical image superimposed with a region display including at least any one of a detection unit region display indicating a region where the X-ray detection unit is arranged in the optical image, an X-ray irradiation field region display indicating a region to which X-rays are irradiated by the X-ray irradiation unit in the optical image, and an X-ray light receiving field region display indicating a region receiving X-rays irradiated by the X-ray irradiation unit in the optical image, or displaying the optical image with the region display hidden, without superimposing the region display on the optical image, to a second mode of displaying the optical image superimposed with the region display based on a position on a body surface of the subject in the real space.

### (Item 2)

The X-ray imaging apparatus according to Item 1,
wherein the first mode is a mode for, when performing position adjustment of the subject, displaying the optical image superimposed with the region display based on the fixed position on the display unit, and
the second mode is a mode for, when performing position adjustment of the X-ray irradiation unit, displaying the optical image superimposed with the region display based on a position on the body surface of the subject in the real space on the display unit.

### (Item 3)

The X-ray imaging apparatus according to Item 1 or 2,
wherein the control unit is configured to, when displaying the optical image in the second mode, perform control to display the optical image superimposed with the region display deformed according to a body thickness of the subject based on a position on the body surface of the subject in the real space on the display unit.

### (Item 4)

The X-ray imaging apparatus according to any one of Items 1 to 3, further comprising:
a light source unit that irradiates visible light to an irradiation field region indicating a region to which X-rays are irradiated by the X-ray irradiation unit,
wherein the input receiving unit is configured to receive an operation input to turn on the light source unit, and
the control unit is configured to perform control to switch a mode for displaying the optical image from the first mode to the second mode based on receiving the operation input to turn on the light source unit.

### (Item 5)

The X-ray imaging apparatus according to Item 4,
wherein the control unit is configured to perform control to superimpose the detection unit region display, the X-ray irradiation field region display, and the X-ray light receiving field region display as the region display on the optical image to display on the display unit when displaying the optical image in the second mode.

### (Item 6)

The X-ray imaging apparatus according to Item 5,
wherein the control unit is configured to make a display aspect of the region display in the first mode and a display aspect of the region display in the second mode mutually different to perform control to superimpose on the optical image and display on the display unit.

### (Item 7)

The X-ray imaging apparatus according to any one of Items 3 to 6,
wherein the control unit is configured to acquire the body thickness of the subject based on any one of the optical image captured by the optical imaging unit, a selection operation of body thickness information of the subject by the operator, and body type information of the subject, and to perform control to display the optical image superimposed with the region display deformed according to the acquired body thickness of the subject based on a position on the body surface of the subject in the real space on the display unit in the second mode.

### (Item 8)

The X-ray imaging apparatus according to any one of Items 1 to 6,
wherein the control unit is configured to, when superimposing the region display on the optical image to display in the first mode, perform control to display the optical image superimposed with the region display based on the fixed position on the display unit, based on position information of the X-ray detection unit or standard body thickness information preset for each imaging region of the subject.

### (Item 9)

The X-ray imaging apparatus according to any one of Items 1 to 7, further comprising:
a holding unit that holds the X-ray irradiation unit,
wherein the optical imaging unit is provided on the holding unit together with the X-ray irradiation unit.

### (Item 10)

The X-ray imaging apparatus according to any one of Items 1 to 9,
wherein the control unit is configured to switch a mode for displaying the region display superimposed on the optical image from the second mode to the first mode, based on the input from the operator received by the input receiving unit, or the optical image.

### (Item 11)

An X-ray imaging apparatus, comprising:
an X-ray irradiation unit including an X-ray tube;
an X-ray detection unit that detects X-rays irradiated from the X-ray irradiation unit and passed through a subject;
an optical imaging unit that captures an optical image of the subject;
a display unit that displays the optical image captured by the optical imaging unit;
a body surface position acquisition unit that acquires a position of a body surface of the subject in a real space;
a control unit that performs control to display the optical image on the display unit; and an input receiving unit that receives an input from an operator,
wherein the control unit is configured to switch from a first mode of, when performing position adjustment, based on the input from the operator received by the input receiving unit or the position of the body surface of the subject acquired by the body surface position acquisition unit, with reference to a predetermined fixed position in the real space, displaying the optical image superimposed with a region display including at least any one of a detection unit region display indicating a region where the X-ray detection unit is arranged in the optical image, an X-ray irradiation field region display indicating a region to which X-rays are irradiated by the X-ray irradiation unit in the optical image, and an X-ray light receiving field region display indicating a region receiving X-rays irradiated by the X-ray irradiation unit in the optical image, or displaying the optical image with the region display hidden, without superimposing the region display on the optical image, to a second mode of displaying the optical image superimposed with the region display based on a position on the body surface of the subject in the real space.

### Description of Reference Numerals

10 X-ray irradiation unit
11 X-ray tube
20 X-ray detection unit
31 Optical imaging unit
32, 301, 402 Optical imaging control unit (control unit)
33a Irradiation field region (region irradiated with X-rays)
40 Holding unit
41 Display operation unit (display unit)
43 Input receiving unit
70 Optical image
80 Region display
81 Detection unit region display
84 X-ray irradiation field region display
87 AEC region display (X-ray light receiving field region display)
100, 300, 400 X-ray imaging apparatus
101 Subject
101a Body surface of subject
102 Body type information of subject
103 Position information of X-ray detection unit
104 Standard body thickness information
401 Optical imaging unit (body surface position acquisition unit)

## Claims

1. An X-ray imaging apparatus comprising:
an X-ray irradiation unit including an X-ray tube;
an X-ray detection unit configured to detect an X-ray emitted from the X-ray irradiation unit and transmitted through a subject;
an optical imaging unit configured to capture an optical image of the subject;
a display unit configured to display the optical image captured by the optical imaging unit;
a control unit configured to perform control to cause the display unit to display the optical image; and
an input receiving unit configured to receive an input from an operator,
wherein the control unit is configured, when performing position adjustment, to perform control, based on an input from an operator received by the input receiving unit, to switch from a first mode to a second mode, the first mode being a mode in which, with reference to a predetermined fixed position in a real space, either the optical image with a region display superimposed thereon is displayed, the region display including at least one of a detection unit region display indicating, in the optical image, a region in which the X-ray detection unit is arranged, an X-ray irradiation field region display indicating, in the optical image, a region irradiated with X-rays by the X-ray irradiation unit, and an X-ray receiving field region display indicating, in the optical image, a region for receiving the X-rays irradiated by the X-ray irradiation unit, or the optical image in which the region display is hidden is displayed without superimposing the region display on the optical image, the second mode being a mode in which the optical image with the region display superimposed thereon is displayed with reference to a position on a body surface of the subject in the real space.

2. The X-ray imaging apparatus according to claim 1,
wherein the first mode is a mode for displaying, on the display unit, the optical image on which the region display is superimposed based on the fixed position, when performing the position adjustment of the subject, and
the second mode is a mode for displaying, on the display unit, the optical image on which the region display is superimposed based on the body surface of the subject in the real space, when performing position adjustment of the X-ray irradiation unit.

3. The X-ray imaging apparatus according to claim 2,
wherein the control unit is configured to, when displaying the optical image in the second mode, perform control to cause the display unit to display the optical image on which the region display deformed according to a body thickness of the subject is superimposed based on the position on the body surface of the subject in the real space.

4. The X-ray imaging apparatus according to claim 3, further comprising a light source unit configured to irradiate visible light to an irradiation field region indicating a region where the X-ray is irradiated by the X-ray irradiation unit,
wherein the input receiving unit is configured to receive an operation input for turning on the light source unit, and
the control unit is configured to perform control to switch a mode for displaying the optical image from the first mode to the second mode based on receiving the operation input for turning on the light source unit.

5. The X-ray imaging apparatus according to claim 4,
wherein the control unit is configured to, when displaying the optical image in the second mode, perform control to cause the display unit to display the optical image on which the detection unit region display, the X-ray irradiation field region display, and the X-ray receiving field region display are superimposed as the region display.

6. The X-ray imaging apparatus according to claim 5,
wherein the control unit is configured to perform control to make a display form of the region display in the first mode and a display form of the region display in the second mode different from each other, and cause the display unit to display the region display superimposed on the optical image.

7. The X-ray imaging apparatus according to claim 3,
wherein the control unit is configured to acquire the body thickness of the subject based on any of the optical image captured by the optical imaging unit, a selection operation of body thickness information of the subject by the operator, and body type information of the subject, and to perform control to cause the display unit to display the optical image on which the region display deformed according to the acquired body thickness of the subject is superimposed based on the position on the body surface of the subject in the real space, in the second mode.

8. The X-ray imaging apparatus according to claim 1,
wherein the control unit is configured to, when displaying the region display superimposed on the optical image in the first mode, perform control to cause the display unit to display the optical image on which the region display is superimposed based on the fixed position, on the basis of position information of the X-ray detection unit or standard body thickness information preset for each imaging region of the subject.

9. The X-ray imaging apparatus according to claim 1, further comprising a holding unit configured to hold the X-ray irradiation unit,
wherein the optical imaging unit is provided in the holding unit together with the X-ray irradiation unit.

10. The X-ray imaging apparatus according to claim 1,
wherein the control unit is configured to switch a mode for displaying the region display superimposed on the optical image from the second mode to the first mode, based on the input of the operator received by the input receiving unit or the optical image.

11. An X-ray imaging apparatus comprising:
an X-ray irradiation unit including an X-ray tube;
an X-ray detection unit configured to detect an X-ray emitted from the X-ray irradiation unit and transmitted through a subject;
an optical imaging unit configured to capture an optical image of the subject;
a display unit configured to display the optical image captured by the optical imaging unit;
a body surface position acquisition unit configured to acquire a position of a body surface of the subject in a real space;
a control unit configured to perform control to cause the display unit to display the optical image; and
an input receiving unit configured to receive an input from an operator,
wherein the control unit is configured, when performing position adjustment, to perform control, based on an input from an operator received by the input receiving unit, to switch from a first mode to a second mode, the first mode being a mode in which, with reference to a predetermined fixed position in a real space, either the optical image with a region display superimposed thereon is displayed, the region display including at least one of a detection unit region display indicating, in the optical image, a region in which the X-ray detection unit is arranged, an X-ray irradiation field region display indicating, in the optical image, a region irradiated with X-rays by the X-ray irradiation unit, and an X-ray receiving field region display indicating, in the optical image, a region for receiving the X-rays irradiated by the X-ray irradiation unit, or the optical image in which the region display is hidden is displayed without superimposing the region display on the optical image, the second mode being a mode in which the optical image with the region display superimposed thereon is displayed with reference to a position on a body surface of the subject in the real space.
